# EUROPEAN PATENT SPECIFICATION

(11) **EP 2 601 603 B1**
(45) Date of publication and mention of the grant of the patent: **26.09.2018**
(21) Application number: 11741460.7
(22) Date of filing: 05.08.2011
(51) Int. Cl.: G06F 19/00

(54) **METHOD FOR AGGREGATING TASK DATA OBJECTS AND FOR PROVIDING AN AGGREGATED VIEW**
VERFAHREN ZUR AGGREGATION VON TASK-DATENOBJEKTEN UND ZUR BEREITSTELLUNG EINER AGGREGIERTEN ANSICHT DAVON
PROCÉDÉ D'AGRÉGATION D'OBJETS DE DONNÉES DE TÂCHE ET DE FOURNITURE D'UNE VUE AGRÉGÉE

(30) Priority: 05.08.2010 EP 10172090
(43) Date of publication of application: 12.06.2013
(73) Proprietor: Roche Diagnostics GmbH, 68305 Mannheim (DE); F. Hoffmann-La Roche AG, 4070 Basel (CH)
(72) Inventor: VON ALLMEN, Bernhard, 5707 Seengen (CH); STEIMLE, Anton, 8630 Rüti (CH); SUTER, Urs, 8049 Zürich (CH)
(74) Representative: Richardt Patentanwälte PartG mbB
(86) International application number: PCT/EP2011/063523
(87) International publication number: WO 2012/017073

(56) References cited:
- WO-A1-2009/106081
- WO-A2-2006/057953
- US-A1- 2006 148 063
- US-A1- 2006 178 776

## Description

### Field of the invention

The present invention relates to an analysis system and method for aggregating task data objects and providing an aggregated view, wherein at least some of the aggregated task data objects are indicative of a laboratory procedure.

### Background and related art

In analytical laboratories, in particular clinical laboratories, a multitude of analyses on biological samples are executed in order to determine physiological and/or biochemical parameters being indicative of a disease, nutrition habits, drug effectiveness and/or organ function.

With the advent of new high-throughput technologies and advancement in lab automation systems, the number and complexity of tasks performed in parallel in a laboratory by a multitude of different lab-devices has tremendously increased. While lab atomization helped to improve the quality of analysis results and to reduce payroll costs, lab automation has not rendered human interaction with automated or semi-automated laboratory workcell systems unnecessary. As lab automation allows performing a multitude of pre-analytical, analytical and/or post-analytical laboratory procedures in parallel by a multitude of different laboratory devices and/or laboratory workcell systems, a growing number of laboratory procedures executed in parallel has to be monitored and controlled.

Lab-devices may fail or run out of reagents or other consumables. Often, a human is required to refill an empty reagent box, to replenish a consumable or to repair a failed lab-device. In addition, in many cases human interpretation of the gathered measurement values is still required. Although fewer lab workers are required to execute diagnostic or analytical tasks, a large number of highly diverse tasks related to monitoring and operating an analysis system and related to evaluating the gathered result still require human intervention.

Prior art laboratory workflow management systems allow the assignment of tasks to be performed in the context of a laboratory workflow to a particular user, thereby helping to clarify responsibilities in monitoring, controlling and/or executing tasks.

US 2009/0094529 A1 describes a clinical application system facilitating completion of clinical workflow tasks. The system allows associating tasks in a clinical workflow with external actors and includes a user interface displaying clinical information to a user. The tasks are represented as links in the user interface. Each of the links triggers access to a corresponding external actor.

US 2009/0099871 A1 describes a multi-screen healthcare information management system which allows a user to simultaneously access, display and manipulate various healthcare information that are logically related on multiple screens without jumping around or entering and exiting various screens. The system allows a physician to write orders while viewing other biomedically relevant data.

US 2003/0045958 A1 describes a method for providing a displayable schedule of tasks. In an embodiment, a list of tasks to be performed by a worker is compiled in response to receiving identification information of a worker and based on a list of service tasks.

US 5832475 disclose a method for performing database queries including GROUP-BY operations, in which aggregate values for attributes are desired for distinct subsets of tuples satisfying a query. US 6996554 discloses a database processor for on-line analytical processing, whereby the processor obtains multi-dimensional aggregates of an input relation.

US 5832475 A discloses a database system and method employing data cube operator for group-by operations.

US 20060178776 A1 discloses systems and methods for the automatic control and scheduling of a staining apparatus for biological samples on slides present within the apparatus. The staining apparatus performs some of the staining tasks on the individual slides in accordance with their respective protocols. The tasks may be prioritized and scheduled for increasing or maximizing the throughput of the slides. The robot scheduler may respond to spontaneous user actions and adaptively schedule or reschedule robot actions.

### Summary of invention

The invention provides for an analysis system for analyzing biological samples, a method and a computer program product as claimed in the respective independent claims. Embodiments of the invention are given in the dependent claims.

Embodiments of the invention to provide for an improved analysis system and an improved computer-implemented method for displaying tasks which need to be performed in order to execute or complete a laboratory workflow.

In a further aspect, embodiments of the invention aim to provide an improved analysis system, an improved computer-implemented method and an improved computer program product for assisting a worker in efficiently executing tasks having been assigned to him or her.

In a further aspect, embodiments of the invention aim to provide an improved analysis system, an improved computer-implemented method and an improved computer program product for assisting a worker in efficiently identifying and/or triggering actions which need to be performed in order to keep one or more monitored lab-devices operative.

The term **'lab-device'** as used herein encompasses any pre-analytical, analytical and/or post-analytical laboratory device which is operable to perform a laboratory workflow step on one or more biological samples. A lab-device can be, for example, a centrifuge, a capping- or decapping unit, a sample storage unit, a conveyor belt, an analyzer, an aliquoter, a photometer or the like. A lab-device can be an analyzer or a receptacle of a sample, an analytical adjuvant material, a reagent, a wash buffer, an auxiliary liquid, a pipette, pipette tip or bulb or the like. A lab-device can also be any decapper device, a sample preparation and distribution system, a post-analytical device, in particular an automated sample storage device, and the like.

The term "**analysis system**" as used herein encompasses any system of lab-devices which are operated and/or monitored collectively. Typically, but not necessarily so, an analysis system comprises one or more analyzers, but the term 'analysis system' may also refer to a system comprising only one or more pre-analytical and/or post-analytical devices. For the sake of simplicity, said systems of lab-devices are herein also referred to as 'analysis systems'.

An '**analyzer**' is a laboratory apparatus that can analyze a reaction of a biological sample with a reagent for obtaining a measurement value. For example, an analyzer can measure light absorption, fluorescence, electrical potential or other physical or chemical characteristics of the reaction to provide the measurement data.

The term '**mobile device**' as used herein encompasses any mobile electronic appliance having an interface for communicating with a server computer, in particular any handheld battery powered mobile appliance, such as a mobile phone, a smart phone, a personal digital assistant (PDA) or another electronic appliance having a wireless interface for establishing a communication link with a server computer such as over a wireless digital cellular telecommunications network or another wireless communication channel.

The term '**drill-down analysis**' as used herein refers to the execution of data processing and data evaluation techniques including, for example, arithmetic and/or statistical calculations on a set of data objects, wherein the execution of said drill-down analysis is triggered by a user navigating among levels of data ranging from the most aggregated (up) to the most detailed (down).

The term '**data aggregation**' as used herein is any process in which information is gathered and grouped together. Data aggregation allows the gathering of information about particular data objects sharing one or more specific attributes.

A '**task**' as used herein encompasses any laboratory procedure to be performed, e.g. on one or more biological samples, by a lab-device. In addition, the term encompasses any operation to be performed on data being received from one or more lab-devices of an analysis system upon processing a biological sample. The term further encompasses any processing step to be executed by a worker on a lab-device in order to preserve or restore the operability of a lab-device. Accordingly, executing a task can comprise executing a pre-analytical, analytical or post-analytical workflow or workflow step by a lab-device, e.g. an analyzer or a centrifuge. Executing a task can also comprise evaluating measurement values obtained on one or more samples by a user, e.g. by means of a GUI, or refilling an empty reagent container of a lab-device by said user.

The term '**task data object**' as used herein encompasses any data object being indicative of a task. In particular, the term refers to data objects being indicative of a laboratory procedure to be performed on one or more biological samples using one or more lab-devices. A task data object can be, for example, an instance of a class object specified in an object oriented programming language such as Java or C#.

A '**consumable**' as used herein is any liquid, material or device component needing a refill or replacement action on a regular basis while operating one of the lab-devices. A consumable can be, for example, a reagent, a calibration liquid or a cuvette for executing optical measurements.

In accordance with embodiments of the invention a method being implemented by an analysis system is provided, the analysis system being used for processing biological samples, the method comprising the steps of:
A) receiving task data objects, each task data object comprising at least one attribute, wherein at least some of the received task data objects are indicative of a laboratory procedure to be performed by at least one lab-device, the at least one lab-device belonging to the analysis system,
B) aggregating at least some of the received task data objects into aggregated task data object groups, wherein all task data objects belonging to the same aggregated task data object group share an attribute value or value range of the at least one attribute, the at least one shared attribute value or value range being indicative of at least one shared step of the task of said attribute's task data object, said shared step being shared by all tasks of the task data objects of said aggregated task data object group,
C) specifying a selectable aggregation GUI element for each of the aggregated task data object groups, said aggregation GUI element representing said aggregated task data object group,
D) displaying the aggregation GUI elements in an aggregated view on a graphical user interface, and
E) upon selection of one of the aggregation GUI elements by a user, selecting the aggregated task data object group represented by the selected aggregation GUI element and automatically providing said user access to program instructions for executing the shared step of said selected aggregated task data object group.

Executing an aggregation steps based on a shared task step may advantageous for the following reason: the user is less disturbed by 'visual noise' as the tasks are not displayed in list form but rather are displayed in the form of a comparatively small number of aggregation GUI elements.

Further, the feature that the aggregation GUI elements are selectable and that a selection of one of the aggregation GUI elements automatically provides the user access to program instructions for executing the shared step of said selected aggregated task data object group may be advantageous, because the user immediately reaches program instructions for executing all aggregated tasks of the selected aggregation GUI element. The shared step can be a step executed at a shared physical location, e.g. a particular building or room or a particular lab-device. Said shared step may likewise relate to program instructions which are shared by all tasks represented by the task data objects of one aggregated task data object group. Said shared program instructions may allow executing or beginning to execute - at one shared physical or logical position within a workflow - a particular step within said workflow. Said shared program instructions may specify a shared workflow execution view, may specify a shared program routine or a shared command for operating some of the lab-devices.

For example, the display of a workflow execution view may be triggered, said view enabling a user to execute a plurality of evaluation tasks by means of one and the same GUI content. Thus, the user does not have to switch between different views for executing the same task. Likewise, said workflow evaluation view may comprise an indication of a plurality of maintenance tasks aggregated according to the shared task step of retrieving consumables for refilling the lab-devices from one particular storage room, thereby enabling a user to go to said storage room only once, thereby picking up all consumables required to fill up a plurality of different lab devices. State of the art systems wherein tasks are presented to a user in list form are less efficient, as the user would simply execute refill actions in accordance with a chronologically or otherwise sorted list of tasks. A 'maintenance' task as used herein encompasses any task of inspecting and/or repairing a lab-device for ensuring that the lab-device remains operative or becomes operative again after a failure, it further encompasses tasks where consumables as reagents, disposable pipette tips, cuvettes and so on are loaded or waste is eliminated.

In accordance with embodiments of the invention, task data objects are received by a server computer. Depending on the embodiment, the task data objects may be received from a single or a plurality of sources. According to some embodiments, a relational database having stored therein one or more task data objects is used as source. Said database may be stored on a storage medium of the server computer or may be stored on a remote database server being accessible by the server computer via a network, e.g. the intranet of a laboratory. The database may also be implemented as a multitude of different databases hosted on one or more different computers. According to embodiments of the invention, the database may also be an integral part of a lab-device belonging to the analysis system.

Depending on the embodiment of the invention, the server computer can be a server or any other kind of standalone processing device being connected to at least one lab-device of the analysis system. According to some embodiments of the invention, the server computer is an integral component of the analysis system. According to further embodiments, the server computer is an integral component of a lab-device of the analysis system. According to still further embodiments, the functionality provided by the server computer can be accessed by other computers, in particular end-user computers. Access is provided e.g. in via the middleware, e.g. a workflow management system, or via the LIS of a laboratory.

According to embodiments of the invention, at least some of the task data objects are received dynamically from a lab-device of an analysis system, the lab-device being connected to the server computer via a network, e.g. an Intranet of a laboratory.

According to embodiments of the invention, at least some of the task data objects represent a test request and are received from a lab-device or from a further computer, said lab-device or said further computer providing a user with means to specify a test request. A test request can be, for example, a request to perform a particular analysis, e.g. an analysis determining the blood glucose concentration of a sample of a particular patient. The test request is represented as task data object and transmitted to the server computer. The further computer may be, for example, a computer of a physician, said computer of the physician being connected to the server computer via a network, e.g. the intranet of the laboratory or the internet.

According to embodiments of the invention, at least some of the task data objects are received dynamically from a lab-device and represent tasks which have to be performed on said lab-device in order to restore or preserve its operability. For example, an analyzer requiring a particular reagent for executing an analysis which has run out of reagent submits a request to refill the container of said reagent to the server computer.

According to further embodiments of the invention, the task data objects received by the server computer from a lab-device are indicative of an error state of said lab-device and/or are indicative of the task required to restore operability of said device. According to further embodiments, the task data objects received by the server computer from the one or more lab-devices comprise additional information on how the indicated task is to be executed by a user. A task data object being indicative of the task to refill a particular reagent for a particular lab-device may comprise, for example, data on the position of the lab-device, e.g. the room number and/or an identifier of the laboratory the lab-device belongs to. Said task data object may in addition or alternatively comprise data on the lot number of the reagent, on the storage room where a new bottle of reagent can be retrieved, on the shelf-life of the reagent etc.

Depending on the embodiment of the invention, receiving one or more task data objects can be executed via a push- or pull method or a combination thereof.

According to embodiments, the server computer retrieves task data objects stored in the relational database via the pull method on a regular basis. In addition, task data objects dynamically generated by a user in the form of e.g. a test request or dynamically generated by a lab-device e.g. in the form of an error or status message may be submitted from the lab-device to the server computer and/or the relational database via the push method.

Program modules managing the data exchange between server computer, the one or more lab-devices and the one or more sources for receiving the task data objects are, according to embodiment of the invention, integrated into the middleware of a laboratory or are integrated into a laboratory information system (LIS).

At least some of the received task data objects are indicative of a laboratory procedure to be performed by at least one lab-device. A laboratory procedure can be any pre-analytical, analytical or post analytical step of a laboratory workflow, including multiple steps of a workflow, to be executed on one or more biological samples. A biological sample can be, for example, a blood or serum sample of a patient. A biological sample may also be a multi-well plate, tissue slides, cell cultures, Chip assays or the like.

Each of the received task data objects comprises one or more attributes. An attribute can be, for example and without limitation:
- the type of the indicated task (e.g. lab-device maintenance, requested analytical tests, evaluation of measurement results);
- an urgency level of the indicated task; an urgency level indicates the urgency and priority of a task. Highly urgent tasks require immediate action of a user in order to preserve operability of the analysis system.
- the time at which the indicated task is to be executed, e.g. a particular date or time;
- an identifier of a physical location where the indicated task is to be executed, whereby said physical location can be, for example:
   - a device-ID being indicative of a lab-device, said lab-device to be used for performing the indicated task,
   - an identifier of a component of a lab-device assigned to the indicated task, e.g. a calibrating unit being integral part of an analyzer,
   - a specification of a place the lab-device is located at, the lab-device to perform the indicated task; the specification can be, for example, a room number and/or an identifier of a laboratory,
   - a specification of a place a biological sample is stored in or can be retrieved from, the biological sample to be processed in the indicated task,
   - a storage-room-ID and/or a storage device-DI of the room or device wherein a reagent used for executing the indicated task is stored,
- an identifier of a set of program instructions for executing the indicated task, whereby said program instructions can be, for example:
   - an identifier being indicative of a GUI pane comprising GUI elements for evaluating analysis results obtained on biological samples,
   - an identifier being indicative of a GUI pane comprising GUI elements displaying messages on hardware failures in one of the at least one lab-device,
   - an identifier being indicative of a GUI pane comprising GUI elements for displaying a manual how a detected hardware error can be fixed,
- a user-ID of a user assigned to the indicated task,
- a role-ID of a user role assigned to the indicated task,
- a group-ID of a user assigned to the indicated task,
- an identifier of the type of the indicated task,
- a pre-analytical, analytical or post-analytical procedure to be performed according to the indicated task, e.g. the determination of the glucose level in a blood sample,
- a type of a reagent used for executing the indicated task,
- a supplier ID of the supplier of a reagent used for executing the indicated task, and
- a patient-ID of the patient providing the biological sample to be processed during the indicated task.

Depending on the embodiment of the invention and on the attribute(s) used for aggregation, the created aggregated task data object groups can be disjoint or overlapping in respect to the task data objects contained therein.

According to embodiments, at least some of the task data object groups respectively comprise an aggregated data value, said aggregated data value being calculated from all task data objects being contained in said aggregated task data object group. The calculation may use all or parts of the data contained in each task data object belonging to said aggregated task data object group. The calculation of the aggregated data value can comprise, for example, executing a mathematical function on one or more data values of all task data objects belonging to an aggregated task data object group, thereby returning an aggregated data value as result. The mathematical function may comprise, for example, the calculation of a minimum or maximum value, the calculation of a sum or any combination of said or another mathematical operation. The calculated data value may also be the number of task data objects contained in a particular aggregated task data object group. Said number may be displayed by the GUI element representing said aggregated task data object group.

According to embodiment, each selectable aggregation GUI element provides the user access to program instructions for executing the tasks indicated by the task data objects represented by said aggregation GUI element. For example, tasks aggregated together into one aggregated task data object group can be validation tasks to be performed on a particular kind of measurement result, e.g. by means of a manual inspection of the obtained measurement values and/or by a visual inspection of plots having been automatically generated based on the measurement values. Accordingly, a shared task step may be the display of one out of a plurality of available workflow execution views which comprises GUI elements enabling a user to evaluate a particular type of measurement value. Said GUI elements can be, for example, text labels, buttons, evaluation functions, statistical graphs of analytical test results and the like. According to another example, the aggregated tasks may also be a set of calibration tasks. A calibration task may comprise the task of calibrating one of the lab-devices, wherein a shared step of the calibration task is executing the calibration at lab-devices located at the same location, e.g., the same room, department or lab. Thus, the user is enabled to execute the tasks more efficiently because all calibration tasks to be executed e.g. in the same room are bundled. A task may also be the maintenance of a plurality of lab devices sharing the same location. Said location may be indicated by the at least one attribute of each task data object, e.g. a room-ID.

A graphical user interface element (GUI element) is specified for each of the aggregated task data object groups whereby an aggregated data representation may be displayed for each of said aggregated task data object group and may be used for specifying the aggregation GUI element of said aggregated group.

A 'view' is a particular type of arrangement of one or more elements on a graphical user interface. An '**aggregated view**' is a view comprising at least one aggregated task data object. Thus, an aggregated view provides the user with an intuitive and quickly comprehensible presentation of all or some of the tasks represented by a multitude of aggregated data objects. Providing an aggregated view can comprise displaying one or more GUI elements respectively representing an aggregated task data object group. In addition, an aggregated view may comprise additional GUI elements showing to the user the number of data objects aggregated, an aggregated priority score, or any other form of aggregated data value. The aggregated data value may be displayed as alphanumerical character, may be encoded by a color schema or may be encoded by using a set of predefined images. The aggregated data value may be displayed, for example, as integral part of the GUI element representing the aggregated task data object group for which said aggregated data value was calculated.

A GUI element is a data object whose attributes specify the shape, layout and/or behavior of an area displayed on a graphical user interface, e.g. a screen. A GUI element can be a standard GUI element such as a button, a text box, a tab, an icon, a text field, a pane, a check-box item or item group or the like. A GUI element can likewise be an image, a displayed alphanumeric character or any combination thereof. An aggregated data value generated in the aggregation step may be used, for specifying a GUI element implies that at least some of the properties of the displayed GUI elements depend on said aggregated data value.

According to embodiments of the invention, the aggregation GUI element comprises a numerical value being indicative of the total number of task data objects of said aggregation data object group.

According to embodiments, each task data object has assigned a user- or group-ID and the method further comprises the step of grouping the tasks data objects into groups sharing the same user-based on this ID, wherein the step of aggregating the task data objects is selectively executed for the group of task data objects having assigned the user- or group- ID of a logged- in user.

According to other embodiments, each task data object has assigned a user- or group-ID and the method further comprises the step of filtering the tasks data objects based on this ID, wherein the step of aggregating the task data objects is selectively executed for the group of task data objects having assigned the user- or group-ID of a logged in user.

According to embodiments of the invention the aggregated GUI element can comprise a numerical value being indicative of the total number of tasks to be executed by a lab-device being identified by a shared device-ID, the shared device-ID being indicative of the shared task step. Alternatively, or in addition, the aggregation GUI element can be an image being indicative of the lab-device or type of the lab-device whose device-id is assigned to the aggregated task data objects, thereby facilitating a user the identification of the lab-device where the tasks indicated by the aggregated task data objects are to be performed. Instead of or in addition to a lab-device-ID, a component-ID of said lab device may be used for aggregation.

According to embodiments, the aggregation GUI element can be indicative of the lab-device-component having assigned said device-component-ID. By highlighting or otherwise optically accentuating said identified device-component where a task needs to be performed, the identification of said lab-device-component by a user for performing the tasks indicated by the task data objects is greatly facilitated.

According to embodiments of the invention, an analyzer comprises one analysis component and one or more reagent components, each reagent component comprising at least one reagent container. In case the reagent container of the first reagent component runs out of reagent, the aggregation of task data objects according to the attribute 'device-component-ID' results in the provision of an aggregated view: the analysis system is represented and displayed on a graphical user interface as a schematic diagram, the schematic diagram representing the analyzer- and reagent components as distinct areas. According to said embodiments, the task of refilling the reagent of the first reagent component is represented as a task data object. Said task data object has assigned the device-component-ID of the first reagent component. Upon aggregating task data objects according to their assigned device-component-IDs, the aggregated task data object groups being indicative of tasks to be performed on or by the first reagent component are represented as GUI element by highlighting the area of the schematic analyzer diagram representing the first reagent component. As a result, the highlighted area of the schematic analyzer diagram provides a user with an aggregated view on tasks which need to be performed on or by the highlighted first reagent component of said analyzer.

The aggregated data value of each aggregated task data object group is used for specifying the GUI element representing said aggregated task data object group. Using the aggregated data value for specifying a GUI element can, but does not necessarily have to, imply displaying an aggregated numerical data value, e.g. the total number of tasks indicated by all task data objects belonging to the aggregated task data object group. Likewise, the value of the aggregated data value can be used to specify a GUI element having a particular design e.g. by using different colors or images. For example, providing an aggregated view of a large number of tasks can comprise displaying GUI elements of a different color compared to providing an aggregated view of a small number of tasks. Likewise, providing an aggregated view of a set of highly urgent tasks can involve the specification of GUI elements having a different visual representation (e.g. color, icon) than the GUI elements representing routine tasks with low priority.

A value range can comprise a range of user-IDs, a range of device-IDs and the like. Likewise, the position of a lab-device can be encoded such that the position-ID of said device comprises both an indicator of the building, of the department and/or the room the device is located in, thereby enabling a coarse-grained or a fine-grained aggregation of task data objects based on an assigned position-ID.

Aggregating data contained in a multitude of task data objects assists a user in performing his tasks more efficiently. In a further advantageous aspect, embodiments of the invention used as a laboratory workflow management system reduce the time to learn because the user does not have to look for and remember which screen to access in order to perform a particular task.

According to further embodiments of the invention, a hierarchical drill-down graph comprising at least two hierarchical levels and comprising one or more first nodes is used for executing the data aggregation. Each first node has assigned a node attribute. The method further comprises the steps of:
- representing each received task data object as a second node of the hierarchical drill-down graph, wherein the aggregating step B comprises:
- determining one of the first nodes as current node of the hierarchical drill-down graph, the current node being a starting point for executing a drill-down analysis, the drill-down analysis being a data aggregation operation executed on the current node and all direct and indirect successor nodes of the current node, wherein when executing the aggregation operation on said nodes, the node attribute of the current node is used in said aggregation operation as aggregation attribute in accordance with step B; and
- calculating, for the aggregated task data object group represented by the current node, an aggregated data value by executing a data aggregation function over all task data objects represented by any of the successor nodes of the current node.

According to embodiments, the first nodes of the drill-down graph specify a predefined graph topology providing for a 'backbone' graph data structure to which a plurality of second nodes respectively representing a task data object are assigned.

According to further embodiments of the invention, the steps of aggregating task data objects can be performed on multiple hierarchical levels of a drill-down graph by selecting a particular node in said drill-down graph. Said embodiments are advantageous, as they provide a user with multiple levels of data aggregation and with means to specify for which node, including its direct and indirect successor nodes, a drill-down analysis shall be executed.

A drill-down graph is a data structure comprising data objects being represented as nodes and edges of a graph. A drill-down graph allows the execution of a drill-down analysis of the data contained in its nodes, wherein the pathways that can be used in the drill-down analysis are specified by the drill-down graph's edges.

According to embodiments of the invention, the drill-down graph comprises a first set of nodes which do not represent task data objects. Each of the received task data object is represented as node and added to the drill-down graph by creating new edges, said new edges connecting at least some of the first nodes with the newly created nodes representing the received task data objects.

According to some embodiments of the invention, all task data objects are represented as leaf nodes of the drill-down graph. According to further embodiments of the invention, at least some of the received task data objects are represented as non-leave nodes. Depending on the embodiment of the invention, the hierarchical drill-down graph comprises two or more hierarchical levels. Each node of the drill-down graph not being a leaf node comprises one or more child nodes. At least some nodes in the drill-down graph can be used as starting point for executing a drill-down analysis. The drill-down analysis is a data aggregation operation executed upon selection of a node as starting node for the drill-down analysis. Said node is referred to as 'current node'. The data contained in the current node and in its direct and indirect successor nodes is aggregated during said drill-down analysis. A 'direct successor node' of a node X is a child node of said node X. An 'indirect successor node' is any node S that can be reached starting from said node X by traversing the graph downwards, wherein S is not a child node of node X.

According to embodiments of the invention, a drill-down analysis executed for a particular current node comprises the steps of:
- aggregating all task data objects being represented by the current node or by a direct or indirect successor node of the current node into aggregated task data object groups, wherein all task data objects belonging to the same aggregated task data object group share an attribute value or value range of the at least one attribute, the at least one shared attribute value or value range being indicative of at least one shared step of the task of said attribute's task data object, said shared step being shared by all tasks of the task data objects of said aggregated task data object group, and
- displaying the specified aggregation GUI elements on a graphical user interface for providing the aggregated view.
According to embodiments, different kinds of attributes are used in different hierarchical levels of the drill-down analysis. For example, the attribute used for aggregating task data objects of a first level of the drill-down graph may be a room-ID shared by a plurality of lab-devices. On a second level of the drill-down graph hierarchy, the attribute used for aggregating groups of task data objects may be a device-ID.

According to further embodiments, a predefined drill-down graph topology specifies the one or more attributes used in each respective drill-down step. This may be advantageous, because the predefined topology determines which kind of attribute or attributes are used in each drill-down operation for executing the aggregation step. Other attributes may be ignored, thereby saving processing power.

According to further embodiments of the invention, the method further comprises the steps of displaying at least one navigation GUI element, wherein the at least one navigation GUI element is a selectable pointer to a node of the drill-down graph; upon selection of said at least one navigation GUI element by a user, using the node being pointed at by the selected navigation GUI element as current node. The at least one navigation GUI element provides a user with the option to specify the starting point for executing a drill-down analysis; and triggering the execution of a drill-down analysis for the used current node. As a result, the user is provided an aggregated view on data contained in task data objects which are represented as direct or indirect successor nodes of the current node used in the drill-down analysis.

According to embodiments of the invention, a 'navigation GUI element' can be any kind of selectable GUI element, e.g. a button, an icon or the like.

According to further embodiments of the invention, the method further comprises the step of displaying two or more navigation GUI elements on the GUI. The displayed navigation GUI elements constitute a navigation path, the navigation GUI elements of said navigation path connecting nodes having already been used as current node in a drill-down analysis. The navigation path allows a user to navigate back and forward between nodes of the drill-down graph by selecting navigation GUI elements pointing to said nodes. The last navigation GUI element in said navigation path is a pointer to a node having been used as current node for executing a drill-down analysis whose resulting aggregated view is currently displayed. According to embodiments of the invention, the first navigation GUI element of said navigation path points to the root node of the drill-down graph.

According to further embodiments, the method further comprises the steps of storing, for each drill-down analysis, at least the aggregated data values in a drill-down history, the drill-down history being stored in a working memory; and selecting a navigation GUI element by a user, the user thereby selecting a new current node. The new current node corresponds to a previously executed drill-down analysis. According to embodiments, the method further comprises the step of loading the stored aggregated data values aggregated in said previously executed drill-down analysis instead of re-executing the drill-down analysis for the selected current node. Said features may be advantageous, as aggregated data values having already been calculated in a previous data aggregation step do not have to be recalculated but can rather be loaded from memory.

A drill-down history is a data structure being stored in the working memory of a computer executing the drill-down analysis. By using said drill-down history, moving forward and backward in the navigation path is significantly accelerated as memory access times of working memories are typically very short and a re-calculation of the drill-down analysis can be avoided by making use of previously calculated results.

According to further embodiments of the invention, the method further comprises the steps of:
- specifying one or more categories,
- representing each of the one or more categories as a selectable category GUI element, wherein said selectable category GUI element can be in particular a tab, but can also be a button, an icon or the like,
- creating, for each category, a corresponding drill-down graph,
- adding each of the one or more nodes representing a received task data object to each of the created one or more drill-down graphs,
- upon selection of one of the one or more category GUI elements by a user, selecting the drill-down graph corresponding to the selected category GUI element, and
- executing a drill-down analysis, wherein the aggregation step is executed according to the graph topology of the selected drill-down graph.

The term '**category**' as used herein refers to an information architecture providing a particular view on the data contained in the received task data objects. Each category corresponds to a drill-down graph. Depending on the embodiment of the invention, the topology of each drill-down graph corresponding to a particular category may be unique, thereby providing a unique graph topology for aggregating and interpreting data contained in the received task data objects.

Using multiple different categories corresponding to multiple different drill-down graphs is particularly advantageous as a user is thereby provided with different entry points to work on the received task data objects and is provided with the option to organize and aggregate tasks according to different schemas. According to embodiments of the invention, each category is selected from a group comprising a 'Routine' category, a 'Lab' category and a 'Utility' category.

The 'Routine' category encompasses data being related to one or more biological samples and data being related to the production of analytical results and/or data providing a user with the option to monitor and/or control the execution of a laboratory procedure on one or more biological sample.

The 'Lab' category can be used to organize data according to one or more lab-devices existing in a laboratory. Views provided according to the 'Lab' category provide a user with the option to monitor and/or control the status of one or more lab-devices, consumables, and/or waste. For example, the filling level of a reagent, error states of a lab-device, or the like can be monitored and the affected device can be started or stopped by the user accordingly.

The 'Utility' category comprises configuration data which can be used for configuring lab-devices, user-roles or user-permissions. The configuration data can also comprise configuration data for of one or more tests to be performed on one or more samples, user management related configuration data and/or log entries.

According to further embodiments of the invention, the method further comprises creating at least one link, the at least one link connecting a first node with a second node, wherein the first node belongs to a first drill-down graph, the first drill-down graph corresponding to a first category, and wherein the second node belongs to the first drill-down graph or to a second drill-down graph, the second drill-down graph corresponding to a second category. In case the second node belongs to the second drill-down graph, the link connects the first and the second drill-down graph and provides a user with the option to navigate between drill-down graphs of different categories.

According to preferred embodiments of the invention, the links are specified by a business analyst, not by an end-user. The usage of links to connect nodes is particular advantageous for the following reasons:
- unlike edges, a link can connect nodes belonging to different drill-down graphs, thereby allowing the user to navigate between different drill-down graphs.
- if a user wants to change context, e.g. wants to view data of one or more groups of task data objects created by aggregating said task data objects according to different drill-down graphs, a link allows a user to switch between different aggregated views, each aggregated view being generated based on the topology of a different drill-down graph.
- a link allows connecting nodes belonging to distant hierarchical levels being separated from each other by one or more other hierarchical levels, thereby allowing a user to skip executing drill-down analyses for each intermediate hierarchical level.
- a link, which is not an edge of one of the drill-down graphs, allows the introduction of circular navigation paths. As a result, a user can be provided with almost unlimited freedom of navigation within or between multiple drill-down graphs while at the same time the generation of cyclic pathways in the drill-down graph which may lead to inconsistencies is prohibited: the topology of a drill-down graph is constituted solely by its nodes and edges, not by the links.

According to further embodiments of the invention, one or more user-IDs and/or role-IDs are assigned to each of the received task data objects. The term 'ID' and 'identifier' are herein used synonymously. A user-ID and/or role-ID of a user are received upon a login of said user. Upon receipt of said user-ID and/or role-ID, only task data objects are used for executing a drill-down analysis which have assigned the received user-ID and/or role-ID.

In particular, a mobile device can be used for the user identification as the mobile device typically signals the user's telephone number when a communication channel is established between mobile device and server computer which can be used for the user's identification. Each user has assigned a user-ID which is an identifier being unique for said user. A user may have assigned a role-ID in addition or instead of a user-ID. A role ID is a unique identifier for a group of one or more users fulfilling a particular role in a laboratory and having assigned role specific rights and duties. Depending on the embodiment of the invention, the user can log into the analysis system via a man-machine interface provided by the server computer, provided by a lab-device connected to the server computer and/or provided by any other computer or a mobile device connected to the server computer via a network. The log-in action may be a log-in action into a software system being interoperable with the analysis system, e.g. a LIS or a middleware component operable to exchange data with the analysis system.

Upon a login-event received from the server computer, an aggregated view is provided to the user, the aggregated view having been created by selectively using only task data objects in the aggregation step which have assigned the user-ID and/or role-ID of the user received upon the log-in event. According to embodiments, the GUI will not display to the logged-in user any tasks he has no permission to execute. This feature may be advantageous, as it reduces the 'visual noise' presented to a user.

According to further embodiments, one or more user-IDs and/or role-IDs are assigned to each of the received task data objects and the method for providing an aggregated view further comprises the steps of: receiving a user-ID and/or role-ID of a user upon a login of said user, grouping the received task data objects in dependence on their respectively assigned user-ID and/or role-ID, and selectively using only task data objects for executing the aggregation step which have assigned the received user-ID and/or role-ID.

According to further embodiments of the invention, each task data object is assigned an urgency level attribute. Some of said embodiments further comprise the step of aggregating task data objects according to the value of the assigned urgency level attribute. In the aggregation step, each aggregated task data object group is assigned an aggregated urgency level value. Said aggregated urgency level value is calculated as the maximum urgency level attribute value of any of the task data objects within said aggregated task data object group. Only GUI elements of those groups of task data objects are displayed whose aggregated urgency level value is above a threshold. According to embodiments, said steps are executed after having determined that no-user is logged-in, thereby ensuring that even in case no user is logged-in an aggregated view on the most urgent tasks to be executed in order to preserve the operability of the analysis system is provided.

According to embodiments, a task data object representing a completed task is automatically or upon an user action flagged as 'completed'. A task data object comprising said flag is not used when executing the aggregation step, thereby further reducing the visual noise.

According to further embodiments of the invention, the method further comprises the steps of specifying a favorite GUI element, the favorite GUI element being a user-specific, selectable pointer to a node within the drill-down graph; and displaying the specified GUI element, wherein upon selection of said favorite GUI element, the execution of a drill-down analysis is executed, wherein in said execution the node being pointed at by the favorite GUI element is used as current node. A 'favorite GUI element' can be, for example, a selectable button, icon or any other selectable GUI element which can be displayed on a graphical user interface. Favorite GUI elements allow users to navigate directly to a certain node in a drill-down graph in order to obtain an aggregated view of task data objects according to a drill-down analysis being based on using said node as current node.

According to further embodiments the task data objects of the selected aggregated task data object group respectively represent a task of inspecting and/or validating a measurement result. Each measurement result is an analysis result or a calibration result. An analysis result is generated by analysing a biological samples. A calibration result is generated analysing a calibration sample. Providing the user access to program instructions for executing the step shared by all said tasks comprises displaying a workflow-execution view. The workflow execution view comprises one or more task execution GUI elements enabling the user to inspect and/or validate said measurement results. The shared step of said selected aggregated task data object group is the step of automatically selecting the workflow execution view from a plurality of workflow execution views for display.

For example, a measurement result can be an analysis result having been generated by the at least one lab device by processing one or more biological samples. According to other examples, each measurement result may be a calibration result having been generated by a calibrator by processing a calibration sample, the calibrator being one of the lab-devices.

According to embodiments, the method further comprises the step of receiving, via the workflow execution view, a confirmation signal being indicative of an approval of one of the measurement results by the user; in case of having received the confirmation signal, said result is automatically forwarded or made available to the LIS or the middleware of the laboratory.

According to embodiments, the at least one lab device is one of a plurality of lab devices and the task data objects of the selected aggregated task data object group respectively represent a task of maintaining one of said plurality of lab devices. Providing the user access to program instructions for executing the step shared by all said tasks comprises displaying a workflow-execution view. The workflow execution view comprises one or more task execution GUI elements, each task execution GUI element comprising information on a current state of one of the lab devices and/or information on how to inspect or repair said lab-device.

According to further embodiments, the shared step of said selected aggregated task data object group is selected from the group comprising:
- executing the maintenance tasks at a shared location, said shared location being shared by all lab-devices whose maintenance tasks are aggregated within the selected aggregated task data object group; for example, the shared location may be indicated by a room-ID, department-ID or lab-ID of the room, department or lab where the respective lab-device is located; and
- selecting the workflow execution view from a plurality of workflow execution views for display; for example, there may be different views specified in an application program, each view comprising a different layout and a different set of GUI elements for executing some maintenance tasks; and retrieving one or more consumables from a shared location for making the respective lab-device operative, said shared location being shared by all lab-devices whose maintenance tasks are aggregated within the selected aggregated task data object group;

According to further embodiments, the shared location is a particular location comprising consumables which need to be retrieved from said location, e.g. a room, a freezer or other storage facility, to replenish empty consumables of a plurality of lab devices. For example, the shared location may be indicated by a room-ID, or freezer-ID where the respective consumables are stored. The method further comprises the step of selecting a workflow execution view from a plurality of workflow execution views for display, said workflow execution view comprising a list of consumables to be retrieved from said shared location.

According to embodiments there may be one first workflow execution view comprising GUI elements designed for displaying an error status without providing means to automatically resolve the problem and there may be second workflow execution views comprising GUI elements enabling the user to manipulate the respective lab-device. Thus, by aggregating all task data objects sharing the display of one particular workflow execution view, a user is empowered to solve multiple maintenance tasks at multiple lab-devices from one single view without having to switch and navigate between different workflow execution views.

According to embodiments, the method further comprises: receiving, via the workflow execution view, a confirmation signal being indicative of a successful maintenance by the user; in case the confirmation signal was received, automatically sending a command to each of the lab-devices for which the confirmation signal was received, thereby automatically activating said lab-device and enabling said lab-device to execute its respective laboratory procedure.

Said features may be advantageous, because the user, maintaining a plurality of lab-devices via one and the same workflow execution view is in addition enabled to re-activate each repaired lab-device e.g. by a click on one of the GUI elements of the workflow execution view. In case the tasks would not have been aggregated in dependence on the shared workflow execution view but rather, for example, chronological, the user would possibly have to switch between multiple different views for maintaining different types of lab-devices, making the interaction between man and machine much more efficient.

For example, a plurality of task data objects may be aggregated according to a shared physical location, e.g. a room comprising a variety of consumables of various lab-devices. A first aggregation GUI element may represent a plurality of tasks for refilling cuvettes, refilling a particular reagent and refilling pipettes, said task being aggregated based on a shared task step of retrieving said consumables from a particular storage room. Said storage room may be represented within the analysis system as an 'unconnected lab-device'. A user clicking on the corresponding aggregation GUI element triggers the display of a workflow execution view comprising a ,shopping list', said list comprising all items to retrieved from said particular room. According to embodiments, a background process monitors the status of the lab devices for calculating a statistical result. Said statistical result is indicative of the average time period until a particular consumable needs to be refilled. This information is used to automatically create a task data object specifying the task of refilling the corresponding consumable within a predefined security time span before the consumable will probably be empty. Thus, embodiments of the invention may not only be able to automatically create task data objects in dependence on received status messages of each of the lab-devices. Rather, said embodiments are in addition able to automatically create task data objects in advance based on automatically gathered, statistical data. Said feature may further improve the efficiency of task aggregation, because items are added to the shopping list which are not yet empty but will probably have to be refilled soon.

According to further embodiments, the at least one lab device is one of a plurality of lab devices and the task data objects of the selected aggregated task data object group respectively represent a task of transporting one or more of the biological samples which have been processed by one of the lab-devices specified in the task data objects of said aggregated task data object group from any of said lab-devices to a destination location. Said destination location can be, for example, a storage unit, a freezer, a fridge, a post-analytical device or the like. The shared step of said selected aggregated task data object group is the step of transporting one or more of the biological samples to a shared destination location. Providing the user access to program instructions for executing the shared step comprises displaying a workflow-execution view. Upon receiving, via the workflow execution view, a confirmation signal being indicative of a confirmation for the destination location by the user, a signal triggering one or more robotic units to automatically execute the transportation tasks of the selected aggregated task data object group is automatically submitted to said robotic units. As a consequence, the robotic units, e.g. a conveyor belt and/or a robotic arm automatically transport each of the biological samples having been processed by any of said lab-devices to the shared destination location. This may be advantageous, because in case the lab-devices are part of an automated, multi-device workcell system, the samples can be collected from multiple lab-devices for a collective transport to the destination location.

According to further embodiments of the invention, the method further comprises the steps of: providing each of the aggregation GUI elements according to a first aggregation GUI element version, each GUI element version corresponding to a particular graphical design, the first GUI element version being adapted to be displayed on a small screen, in particular a screen of a mobile user device, wherein each GUI element is a pointer to one of the first nodes in the drill-down graph; displaying the aggregated view comprising the aggregation GUI elements in a first pane, the first pane approximately being of screen size; and displaying a second pane being approximately of screen size, the second pane replacing the first pane. The second pane comprises one or more further aggregation GUI elements, or comprises one or more task GUI elements. Each task GUI element represents a task data object of an aggregated task data object group represented by one of the aggregation GUI elements of the first pane. Upon selection of any of the task GUI elements, instructions for executing the shared step of said one aggregated task data object group are executed.

A GUI element version corresponding to a particular graphical design is a specification of the size, shape and other features determining the appearance and dynamic behavior of a GUI element on a graphical user interface.

Said embodiments are advantageous as they provide an aggregated view of data which is particularly adapted to be displayed on a small screen, e.g. of a mobile device of a user such as a smart phone. Some of said embodiments can be used as emergency systems allowing the notification of a user with appropriate permissions and skills, e.g. a lab technician, whenever a highly urgent tasks needs to be executed immediately in order to preserve the operability of an analysis system.

According to further embodiments, the content of the second pane is constructed by executing the following steps: upon selection of one of the aggregation GUI elements in the first pane, selecting one of the first nodes of the drill-down graph as current node; executing a drill-down analysis, the drill-down analysis being a data aggregation operation executed on the current node and all direct and indirect successor nodes of the current node, wherein when executing the aggregation operation on said nodes, the node attribute of the current node is used in said aggregation operation as aggregation attribute in accordance with step B, wherein one or more further aggregation task data object groups are created; and representing each of the further aggregated task data object groups by one of the further aggregation GUI elements. This may be advantageous, because the user is empowered, by selecting one of the first aggregation GUI elements, to execute a drill-down analysis and receive as result one or more further aggregation GUI elements which may represent a plurality of task data objects having been aggregated for one of the child nodes of the node corresponding to the selected first aggregation GUI element. In case it is not possible to further drill-down into the graph because bottom of the tree hierarchy is reached, the single task data objects may be represented and displayed as selectable task GUI elements on the second pane. Upon selection of said task GUI element by the user, no further drill-down is executed, but rather, the execution of instructions for executing the task represented by the selected task data object is triggered.

According to embodiments of the invention, the server computer continuously monitors status information of the lab-devices belonging to an analysis system. In dependence on the status information, one or more task data objects are created and received by the server computer. The step of aggregating task data objects is performed by using at least an urgency level attribute assigned to each of the received task data objects as attribute for the aggregation step. In case the aggregated urgency level value assigned to any of the groups of task data objects exceeds a threshold value, one or more users are notified of the urgent tasks. The notification is executed by the server computer sending a specification of an aggregated view on said urgent tasks to the mobile device of a user. Said user can be a default user, e.g. the responsible lab technician. Said user can according to further embodiments likewise be a user who is identified by a user-ID, said user-ID being assigned to each of the aggregated task data objects.

As the size of a mobile device is usually small, it is advantageous to display the results of a drill-down analysis according to a first display mode, the first display mode using panes being approximately of screen size, the first display mode using GUI elements corresponding to a first version of GUI elements. Said display mode can also be considered as 'full screen mode'.

A drill-down analysis is executed upon selecting a GUI element of the first pane. The aggregated data obtained as a result of said drill-down analysis is displayed on a second pane. The first pane and the second pane cannot be viewed by the user at the same time. Rather, the second pane replaces the first pane.

As the size of screens used in current computer systems and/or current analysis systems is usually large enough to allow the simultaneous display of multiple GUI elements at the same time without confusing the user, it is advantageous to display the results of a drill-down analysis on said graphical user interfaces according to a second display mode. The second display mode uses panes being approximately of half screen size. Said display mode can also be considered as 'detailed display mode'. GUI elements belonging to a second version of GUI elements are of appropriate size to be displayed on a medium sized screen such as a screen of a current computer system. A drill-down analysis is executed upon selecting a GUI element of the first pane. The aggregated data obtained as a result of said drill-down analysis is displayed on a second pane. The first pane and the second pane can be viewed by the user according to the second display mode at the same time.

According to further embodiments of the invention, the method further comprises the steps of:
- providing each of the GUI elements according to a second GUI element version, a GUI element version corresponding to a particular graphical design, the second GUI element version being adapted to be displayed on a medium-sized screen, in particular a screen of a computer system or a screen of a lab-device, wherein each GUI element is a pointer to a node in the drill-down graph,
- displaying first GUI elements in a first pane, the first pane approximately being of half screen size,
- upon selection of one of said first GUI elements, using the node being pointed at by the selected GUI element as current node and executing a drill-down analysis by executing steps B-E,
   wherein the GUI elements displayed in step E are second GUI elements, said second GUI elements being displayed in a second pane, the second pane approximately being of half screen size, the first pane and the second pane being displayed at the same time.

According to further embodiments of the invention, previously described embodiments of said method for providing an aggregated view are implemented by an analysis system for analyzing biological samples, the analysis system comprising one or more lab-devices. According to further embodiments of the invention, the method comprises the steps of identifying a user by one of said one or more lab-devices, sending of a user identifier of the identified user and a device identifier of the one of the first and second lab-devices to a server computer, determining a task to be executed by the identified user by the server computer, the server computer thereby using the user identifier and the device identifier, and sending a signal comprising task data objects being indicative of the determined task from the server computer to the lab-device.

According to embodiments of the invention, an aggregation threshold can be specified, e.g. by a business analyst. An aggregation threshold is the minimum number of task data objects that need to be contained within a task data object group in order to determine an aggregated data value and to use said data value for specifying a GUI element representing an aggregated task data object group. According to said embodiments, the number of task data objects in a current task data object group is determined. In case the determined number exceeds the aggregation threshold, a drill-down analysis is executed and a user is provided with an aggregated view. In case it is determined that the current number of task data objects does not exceed the aggregation threshold, each single task data object is graphically represented in the form of a separate GUI element.

According to still further embodiments of the invention, one or more of the GUI elements representing an aggregated task data object group are link GUI elements displayed in a separate area of the screen which is herein referred to as 'tasks area'. Each aggregation element provides a user with access to program instructions for performing the one or more tasks represented by said aggregation GUI element. For example, if 12 task data objects each indicate the task of manually evaluating the plausibility of a measurement value obtained as analysis result, an aggregation GUI element representing said 12 aggregated task data objects comprises a link which, when selected, triggers the display of a more detailed view, said view containing data required for executing said evaluation.

For example, upon selection of said aggregation GUI element, calibration data of the analytical device used for obtaining the measurement values is displayed, said calibration data being required for evaluating the plausibility of an obtained measurement value.

According to further embodiments, selecting an aggregation GUI element by a user triggers the execution of a laboratory procedure on one or more samples by a lab-device and/or triggers the display of one or more selectable GUI elements for controlling, e.g. starting and/or stopping said lab-device.

According to embodiments of the invention, each task data object comprises at least a first and a second attribute. In case a first current node is selected, the first attribute is used for executing a first drill-down analysis comprising the execution of steps B-E. In case a second current node is selected, the second attribute is used for executing a second drill-down analysis comprising the execution of steps B-E, the first and the second attribute not being identical.

For example, during the execution of a first grill-down analysis, a room-ID may be used as attribute for aggregating task data objects. In a second drill-down analysis executed e.g. for a particular group of task data objects all having been assigned to a particular room-ID, a device-ID can be used as attribute for aggregating all task data objects having been assigned said room-ID. Depending on the embodiment of the invention, the attribute or attributes used in the aggregation step may be predefined, specified by a business-analyst and/or specified by an end-user. In addition, one or more grouping steps based, for example, on a user-ID assigned to the task data objects may be executed while executing the drill-down analysis, thereby further reducing the visual noise.

According to embodiments of the invention, the status of the at least one lab-device is monitored by the server computer. The server computer dynamically creates or receives new task data objects and/or modifies or deletes at least some of the received task data objects in dependence on the received status information. Upon creating, modifying or deleting a received task data object the re-execution of a drill-down analysis is triggered. As a result of said re-execution, a dynamically updated, aggregated view is provided to the user.

In accordance with further embodiments of the invention an analysis system for analyzing biological samples is provided, the analysis system comprising: at least one lab-device; a server computer having a server interface component for receiving task data objects, each task data object comprising at least one attribute, wherein at least some of the received task data objects are indicative of a laboratory procedure to be performed by the at least one lab-device. The server computer comprises: a processing component for aggregating at least some of the received task data objects into aggregated task data object groups, wherein all task data objects belonging to the same aggregated task data object group share the same attribute value or attribute value range of the at least one attribute, the at least one shared attribute being indicative of at least one shared step of the task of said attribute's task data object, said shared step being shared by all tasks of the task data objects of said aggregated task data object group; specifying a selectable aggregation GUI element for each of the aggregated task data object groups, said aggregation GUI element representing said aggregated task data object group; displaying the aggregation GUI elements in an aggregated view; upon selection of one of the aggregation GUI elements by a user, selecting the aggregated task data object group represented by the selected aggregation GUI element and automatically providing said user access to program instructions for executing the shared step of said selected aggregated task data object group.
The server computer system further comprises a graphical user interface for displaying the specified aggregation GUI elements for providing the aggregated view.

Depending on the embodiment, the graphical user interface can be part of a lab-device, can be part of the server computer system, and/or can be part of any other processing device being connected to the server computer via a network. Said other processing device can be, for example, a mobile device of a user or a computer connected to the intranet of the laboratory.

According to further embodiments of the invention, a device-ID is assigned to at least some of the received task data objects, the device-ID being indicative of one of the at least one lab-devices. At least one lab-device belonging to the analysis system comprises a device identification component for identifying said lab-device at the server computer via the lab-device's device-ID. According to said embodiments, the server computer comprises a service interface component. The server interface component is operable to send a signal to the identified lab-device, the signal comprising data having been aggregated on all received task data objects to which was assigned the device-ID of the identified lab-device. As a result, a user working on said lab-device can constantly be kept up to date regarding the tasks that need to be executed in the laboratory, in particular urgent tasks.

According to further embodiments of the invention, said device-ID is indicative of a mobile device of the user, the server computer is operable to identify the mobile device via its device-ID, e.g. the mobile number of the user. The server interface component is operable to send a signal to the identified mobile device, the signal comprising data having been aggregated on all received task data objects to which was assigned the user-ID of the user of the identified mobile device. As a result, a user can constantly be kept up to date regarding the tasks that need to be executed in the laboratory, in particular urgent tasks, via his mobile device.

According to further embodiments of the invention, a user-ID and/or role-ID is assigned to at least some of the received task data objects, each user-ID being indicative of one user, each role-ID being indicative of a role assigned to at least one user. The at least one lab-device comprises a user identification component for identifying a user by the user's user-ID and/or role-ID and an interface component for sending the user-ID and/or role-ID of the identified user. The server computer comprises a service interface component, wherein the server interface component is operable to send a signal to the lab-device identified by the device-ID, the signal comprising data aggregated on received task data objects to which is assigned the user-ID and/or role-ID of a user identified by the lab-device. According to further embodiments, the server computer is in addition operable to send said signal to a mobile device of the identified user.

According to still further embodiments, the user can identify himself at the server computer via a graphical user interface provided by the server computer. Said graphical user interface can be a screen of the server computer or can be a monitor screen of a further computer system, the further computer system being connected to the server computer via a network, e.g. the intranet of a laboratory.

In accordance with further embodiments of the invention an analysis system for analyzing biological samples is provided comprising at least one lab-device, the at least one of the lab-device having a user identification component for identifying a user, a device identification component for identifying the lab-device, and an interface component for sending a user identifier of the identified user and a device identifier of the lab-device, and a server computer having a server interface component for receiving the user identifier and the device identifier, a processing component for determining a task to be executed by the identified user, wherein the server interface component is operable to send a signal to the lab-device identified by the device-ID via the service interface component, the signal comprising data aggregated on received task data objects which have assigned the user-ID and/or role-ID of the user identified by the lab-device. Embodiments of the invention are advantageous as the user is informed regarding one or more tasks that need to be executed by that user by the server computer upon logging in using the at least one lab-device. This enables a highly convenient and efficient operation of the analysis system.

In accordance with an embodiment of the invention completion of the execution of a task is signalled to the server computer. For example, the user can use a lab-device for execution of the determined task and then enter an acknowledgement into said lab-device such that completion of the execution is signalled from the lab-device to the server computer. In accordance with an embodiment of the invention the server computer comprises a database for assigning a first set of tasks to the first lab-device and for assigning a second set of tasks to the second lab-device. The processing component of the server computer generates a message when the determined task is not assigned to the identified lab-device in the database.

According to further embodiments of the invention, the method comprises the steps of identifying a user by a lab-device, sending a user identifier of the identified user and a device identifier of the lab-device to a server computer, determining at least one task data object by the server computer using the user identifier and the device identifier, the at least one task data object being indicative of the determined task , and sending a signal comprising the task data objects from the server computer to the one of the lab-devices.

In accordance with an embodiment of the invention the mobile device of the analysis system receives a signal comprising task data objects being indicative of the at least one determined task and also being indicative of the lab-device to be used for execution of this task. In addition the task data objects are indicative of the position of the lab-device to be used for execution. The position information can comprise an indication of the geographic and/or topological location of the lab-device. For example, the position information is provided as a laboratory room number for indicating the laboratory room in which the lab-device is located.

In accordance with an embodiment of the invention the analysis system further comprises at least one mobile device. The mobile device can receive the signal being indicative of the determined task. This signal is also indicative of one of the lab-devices to be used for execution of the determined task. This is advantageous as the integration of at least one mobile device into the analysis system allows ubiquitous operation of the analysis system. In accordance with an embodiment of the invention the task data objects contained in the signal received from the server are indicative of a priority of the tasks. In response to receipt of the signal by the mobile device or the lab-device the tasks are aggregated and an aggregated urgency level is calculated for each aggregated task data object group, the aggregated task data objects being represented by a displayed GUI element only in case the aggregated urgency level of an aggregated task data object group exceeds a threshold value. According to further embodiments of the invention, the mobile device upon completion of at least one determined task sends a message indicating completion of said task to the server computer.

### Brief description of the drawings

In the following, embodiments of the invention are explained in greater detail by way of example, only making reference to the drawings in which:
- Figure 1: is a flow chart of a method providing an aggregated view,
- Figure 2: depicts a screenshot of a graphical user interface (GUI) when no user is logged in,
- Figure 3: depicts a screenshot of a login dialog provided to a user,
- Figure 4a: is a schematic drawing of an overview pane,
- Figure 4b: depicts the overview pane displaying user specific data,
- Figures 5a: depicts an overview on available lab-devices displayed upon selection of the 'Lab' category tab,
- Figure 5b: depicts an aggregated view on consumables used by the lab-device 'Cavallo',
- Figures 6a-6c: illustrate the dynamic specification and aggregated display of tasks,
- Figure 7a: depicts an aggregated view displayed according to a first display mode,
- Figure 7b: depicts an aggregated view generated as the result of a drill-down analysis to the user according to a second display mode,
- Figure 8a: depicts the screen of a mobile device displaying a first pane according to a first display mode, the first pane comprising a tasks pane and a favorites pane,
- Figure 8b: depicts the screen of the mobile device displaying a second pane according to the first display mode, the second pane providing an aggregated view generated as the result of a drill-down analysis,
- Figure 9: depicts parts of two different drill-down graphs corresponding to the categories 'Routine' and 'Lab', the drill-down graphs being connected via a link,
- Figure 10a: is an illustration of the drill-down graph corresponding to the category 'Routine',
- Figure 10b: illustrates the relation between navigational path displayed to the user and the topology of the corresponding drill-down graph,
- Figure 11: is a block diagram of an embodiment of an analysis system of the invention,
- Figure 12: is a block diagram of an embodiment of an analysis system where the task of refilling a reagent is signalled to a user,
- Figure 13: is a screenshot displaying 4 GUI elements respectively representing a different aggregated task data object group,
- Figure 14: is a screenshot of two window panes displaying and /or executing maintenance tasks, and
- Figure 15: is a screenshot of two window panes for displaying and/or executing supply related tasks.

### Detailed description

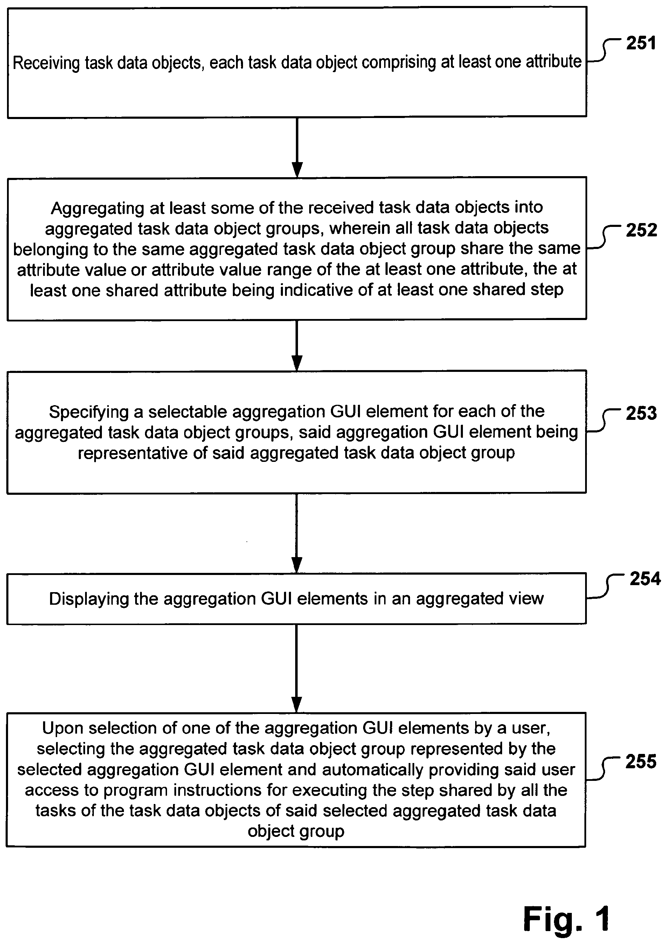

Figure 1 is a flowchart of a method providing an aggregated view to a user. In step 251 task data objects are received, e.g. by a server computer. At least some of the received task data objects are indicative of a laboratory procedure to be performed by the at least one lab-device. In step 252 the received task data objects are aggregated into aggregated task data object groups. All task data objects belonging to the same aggregated task data object group share the same attribute value or attribute value range of at least one attribute. Said at least one shared attribute is indicative of at least one step which is shared by all tasks represented by the respective task data objects of said aggregated task data object group. In step 253, a selectable aggregation GUI element is specified for each of the aggregated task data object groups. Said aggregation GUI element is representative of the aggregated task data object group it represents. In step 254, the one or more aggregation GUI elements are displayed in an aggregation view. Then, in step 255, upon selection of one of the aggregation GUI elements by a user, the aggregated task data object group being represented by the selected aggregation GUI element is selected. The user is automatically provided access to program instructions for executing the step shared by all of the tasks of the task data objects of said selected aggregated task data object group.

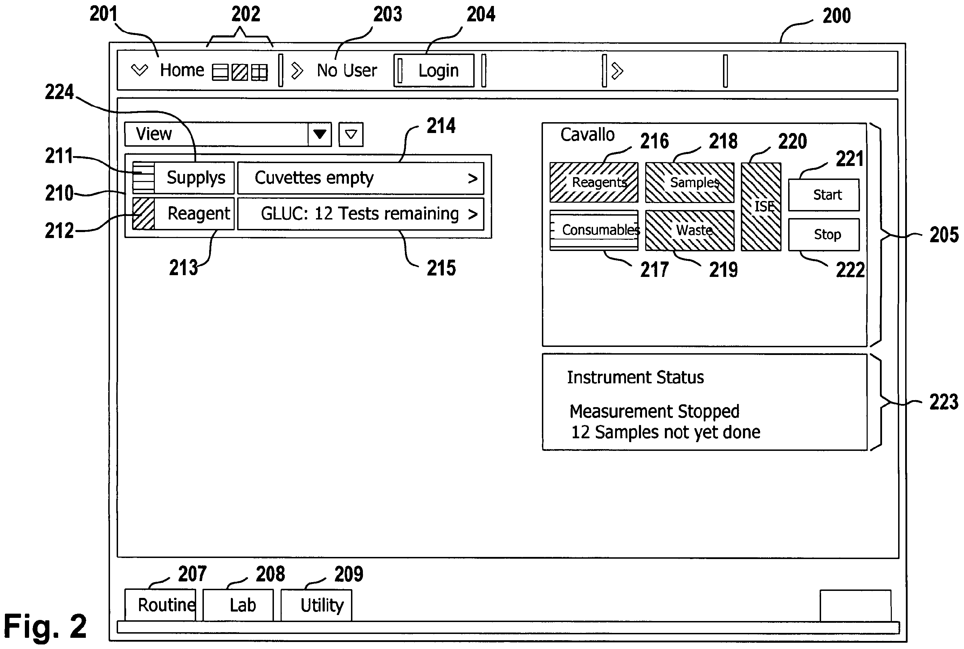

Figure 2 depicts a screenshot of a graphical user interface (GUI) when no user is logged in. The GUI element 203 indicates that currently no user is logged in. The selectable GUI element 204 triggers, upon selection, the display of a log-in dialog window 300 to the user.

The overview pane 201 comprises a tasks area 210, a lab-device area 205 and a lab-device status area 223. Lab-device area 205 displays information on the lab-device named 'Cavallo' in greater detail. Cavallo comprises multiple logical, functional and/or physical subunits: a reagents subunit represented by GUI element 216 comprises one or more reagents for executing a particular analysis; consumables subunit represented by GUI element 217 comprises consumables such as disposable cuvettes or pipette tips; waste subunit is represented by GUI element 219 and a samples subunit for receiving and/or storing biological samples is represented by GUI element 218. Selectable GUI elements 221, 222 are displayed and allow a user to start or stop the lab-device Cavallo.

The GUI elements 221, 222 allow a user to control the operation of the lab-device via the GUI in dependence on the dynamically displayed status information on the lab-device and/or tasks to be performed by said lab-device. The lab-device status area 223 provides a user with an aggregated view on tasks to be performed by one or more lab-devices or by one or more subunits of a lab-device. If one or more tasks needs to be performed by or in respect to one of said subunits, said subunit is highlighted. In the depicted embodiment, the GUI element 217 representing the "consumables" subunit comprising the empty cuvette container is assigned a red colour. Said colour indicates that immediate intervention of a user is necessary in order to restore or preserve operability of the analysis system. In case the server computer determines that further tasks need to be performed not immediately but in the near future, said tasks are indicated by another colour, e.g. yellow. According to the embodiment depicted in Figure 2, the reagents subunit comprises enough reagent for performing the scheduled analyses but needs to be refilled soon. According to embodiments of the invention, the discrimination between tasks to be executed immediately and tasks to be executed in the near future can be specified by using threshold values, e.g. threshold values for a particular period of time until a consumable will be used up, a particular number of samples which can still be processed given a particular amount of consumables etc.

Tasks which have to be executed immediately in order to preserve or restore operability of the analysis system are referred to as tasks of highest priority. According to the depicted embodiment, the highest priority is indicated by red colour. Tasks which need to be executed soon but not immediately are indicated by a second colour, e.g. yellow. Said second colour represents a second urgency level. Tasks which can be executed by a user at any time are indicated by a third colour, e.g. grey or green, said third colour representing the lowest urgency level.

In the depicted embodiment, the one or more tasks to be executed immediately in respect to the consumables subunit are indicated by displaying a GUI element 217 representing the consumables subunit of "Cavallo" according to the colour indicating the highest urgency level. The corresponding one or more tasks 214 displayed in the in the tasks area 210 in the form of an aggregation GUI element comprises a coloured area 211 which is assigned the colour of the highest urgency level. The area 212 in the tasks area 210 and the "reagents" area 216 in the lab-device area 205 are assigned the colour representing the second urgency level (yellow).

According to the depicted embodiment, each task depicted in the tasks area 210 further comprises a text 224, 213 indicating the type of the aggregated tasks, e.g. "Supplies" or "Reagent" in case the aggregated task data objects relate to replenishing supplies or refilling reagents.

The tasks area 210 displays data aggregated on one or more tasks. GUI element 214, for example, is an aggregation GUI element indicating that the cuvette container of the lab-device Cavallo is empty and needs to be refilled. Refilling the cuvette container may relate to one single task or a multitude of tasks. For example, if the cuvette container comprises multiple different types of cuvettes which have to be replenished and have to be retrieved from different storage rooms, a multitude of task data objects indicating said tasks would be dynamically specified. GUI 200 provides the user with an aggregated view on one or more tasks to be executed by or in respect to Cavallo, thereby hiding details such as the type of the cuvettes to be replenished. As a result, the user is provided with a quickly comprehensible overview on the system state and urgent tasks. The aggregation GUI element 215 provides an aggregated view on 12 tasks which relate to 12 remaining glucose tests which could not be executed due to the unavailability of cuvettes. The aggregated view on tasks is highly advantageous, because in the context of a laboratory running large screens a list of tests to be performed on a set of samples may comprise several hundred or thousand items whose display in the form of a non-aggregated task list would disturb the user.

The group of squares 202 indicates the urgency level of the dynamically specified task data objects, the urgency level of a task data object representing the priority of the task indicated by said task data object. In case one or more tasks having assigned the highest urgency level are received by the server computer, the square at the left side turns into the colour of the highest urgency level, e.g. red. In case one or more tasks having assigned the second urgency level were determined, the central square turns into the colour of the second urgency level, e.g. yellow.

The lab-device status area 223 displays information on the current status of the lab-device displayed in lab-device area 205. Lab-device status area 223 in the depicted example indicates that all measurements were stopped and 12 biological samples still need to be processed by Cavallo.

Selectable GUI element "Routine" 207 is a tab element which represents a first category "Routine" and provides access to a corresponding drill-down graph. Likewise, selectable GUI elements "Lab" 208 and "Utility" 209 represent a second and third category, the second category corresponding to a second drill-down graph and the third category corresponding to a third drill-down graph. By selecting one of the selectable category GUI elements 207, 208, 209, a user can specify according to which drill-down graph a drill-down analysis is to be executed. Each task is indicated by a task data object dynamically received by the server computer.

The overview pane 201, herein also referred to as "home" pane, can display aggregation GUI elements representing aggregated task data objects also in case no user is logged into the analysis system. In case the analysis system comprises multiple lab-devices and tasks having assigned a high priority urgency level are assigned to more than one lab-device, the lab-device area 205 may also display multiple different lab-devices. In this case, the whole lab-device is colour-encoded according to the urgency level colour schema explained previously. The main function of the overview pane 201 is to inform a user irrespective of his role and assigned responsibilities on task which have to be immediately executed in order to keep the analysis system running.

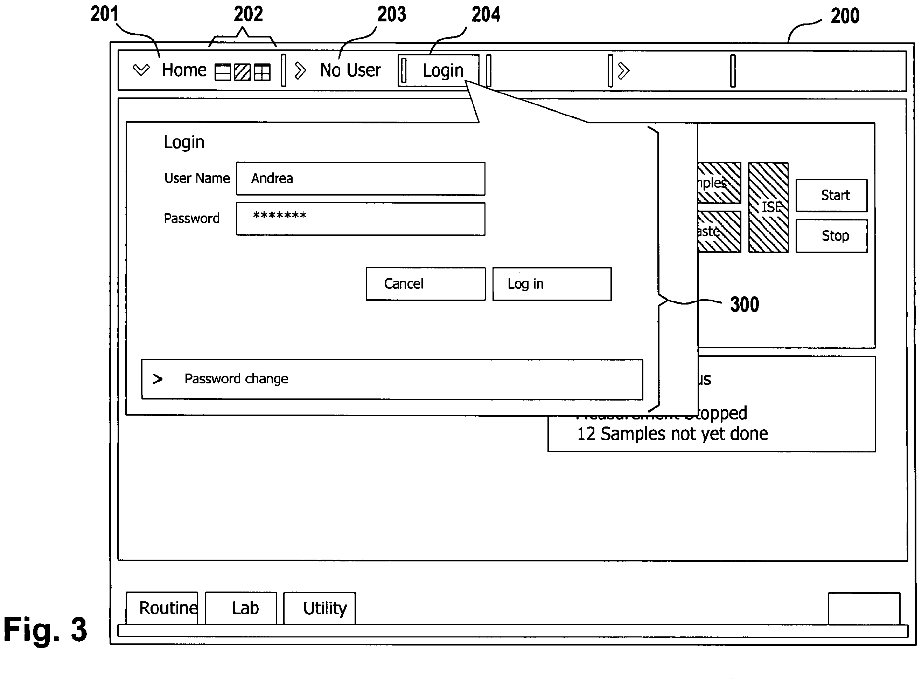

Figure 3 is a screenshot of a login dialog provided to a user e.g. upon selecting the login GUI element 204.

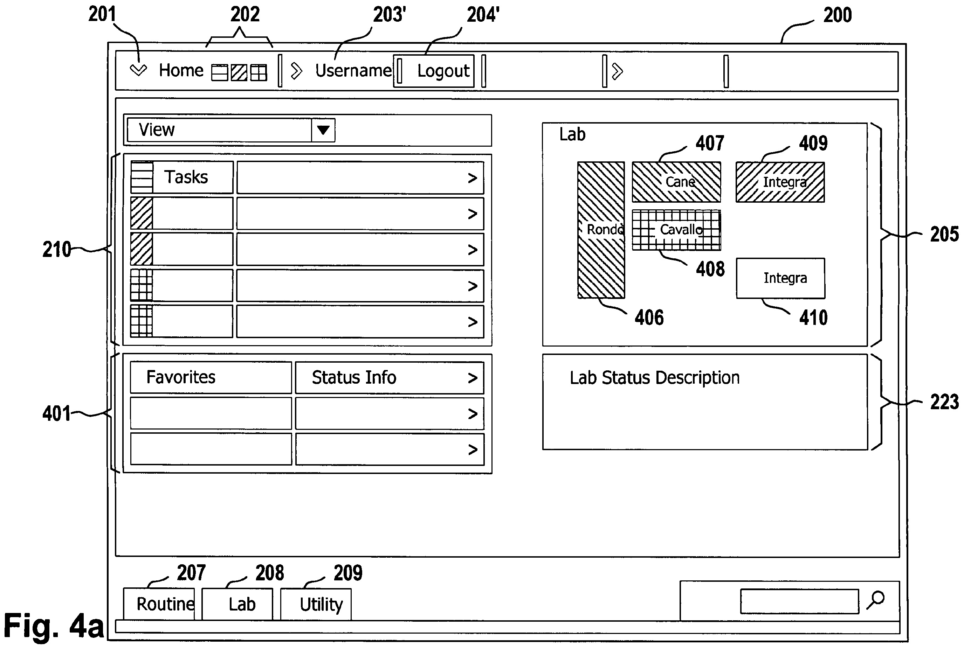

Figure 4a is a schematic drawing of an overview pane displayed when a user has successfully logged into the analysis system. The username of the logged-in user is displayed in GUI element 203'. Upon selection of GUI element 204', the user can log out again. Upon a successful login of a user, the overview pane 201 displayed to a logged-off user as described for figure 2 is complemented with user profile specific information, in particular user-specific tasks and favorites. The favorites pane 401 is a 2-column table comprising user-defined favorites in the first column and dynamically retrieved status information on each favorite item in the second column. The lab-device pane 205 displays a set of GUI elements 406-410 representing lab-devices "Rondo", "Cane", "Cavallo", "Integra 1" and "Integra 2" , said lab-devices being monitored by the server computer.

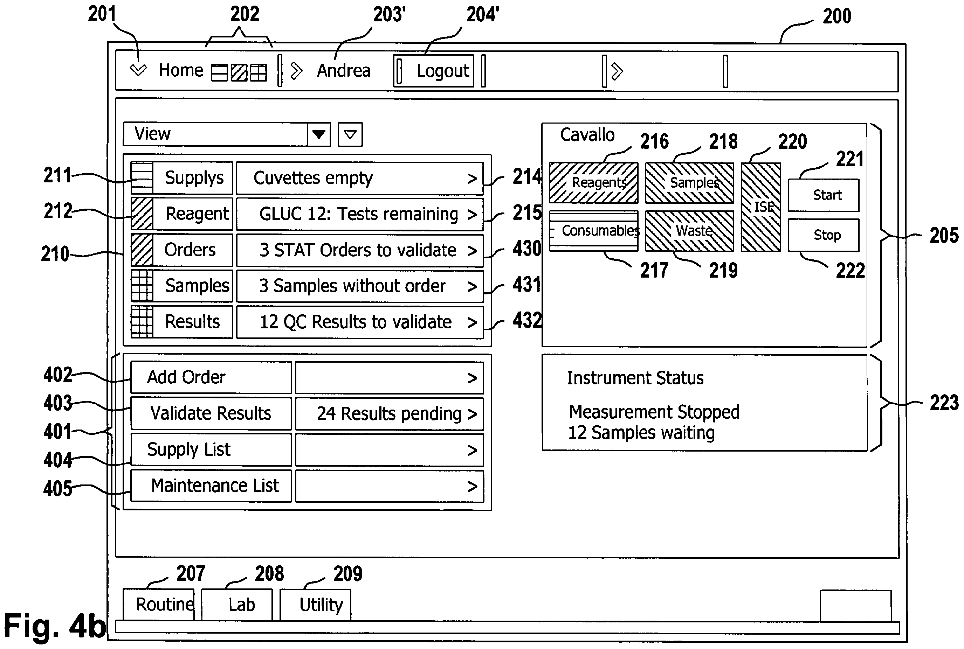

Figure 4b depicts the overview pane after its complementation with user-specific information. Tasks area 205 comprises GUI elements 430, 431 and 432, each providing an aggregated view on one or more user specific tasks. Said GUI elements are displayed in addition to the GUI elements 214, 215 providing an aggregated view on one or more tasks having assigned a high urgency level. The aggregated view provided to a logged-in user by the tasks area 205 urges a user to handle tasks having a high priority level before performing routine tasks.

The favorites pane 401 displays 4 favorites GUI elements 402-405 having been specified by the logged-in user. If available, status information for each favorite item is displayed. Each favorite GUI element represents a particular node in a drill-down graph, thereby representing also a particular aggregated view of task data objects. Each favorite GUI element 402-405 is a selectable GUI element allowing a user to navigate to aggregated view of task data objects represented by the selected favorite GUI element. A user can save time by directly selecting favorite GUI element 403 instead of traversing a drill-down graph until a node and a corresponding aggregated view for evaluating analysis results is reached.

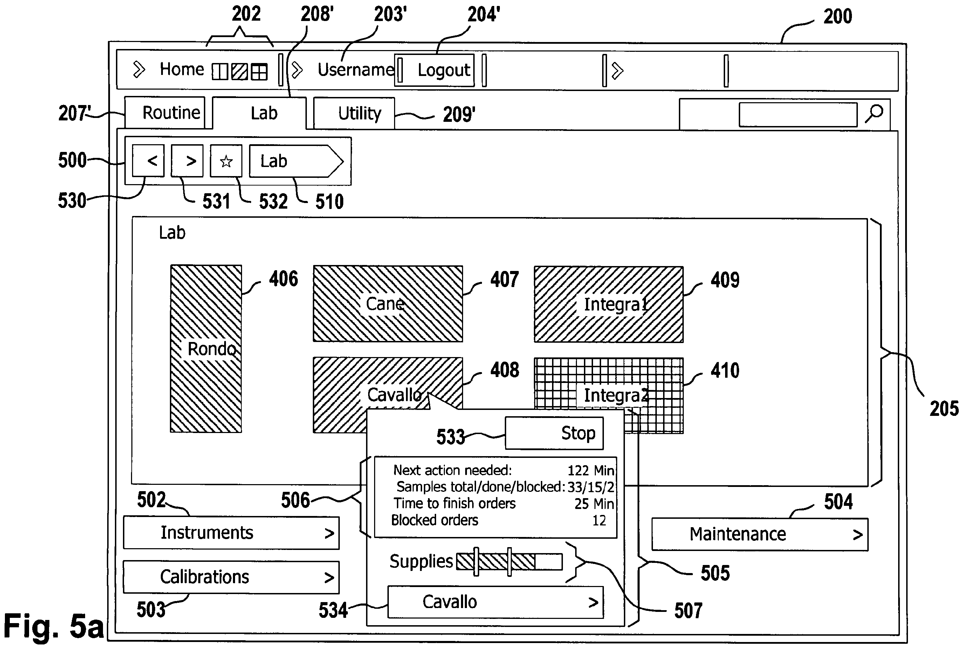

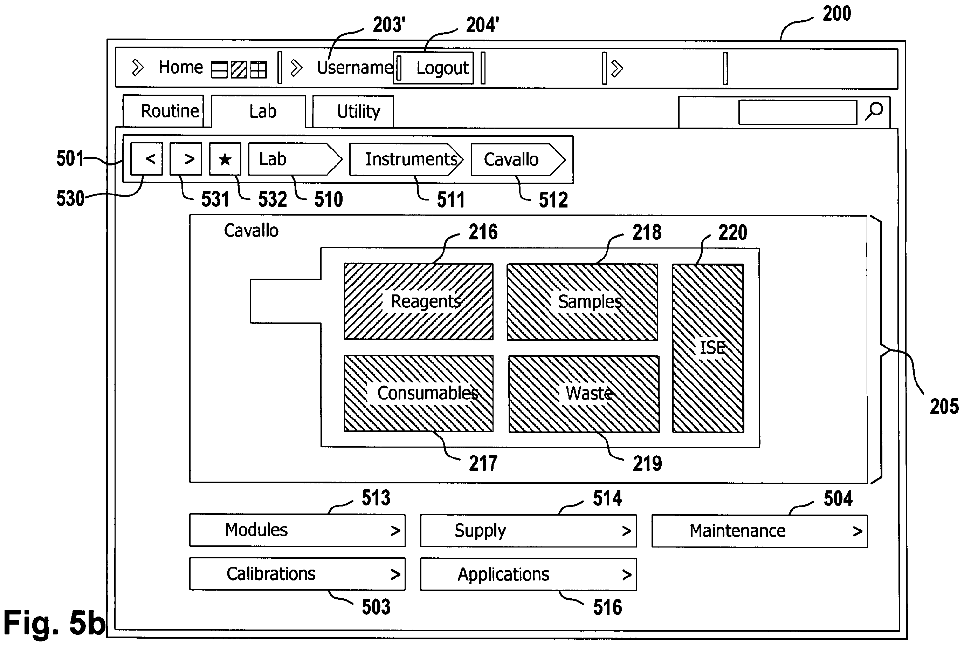

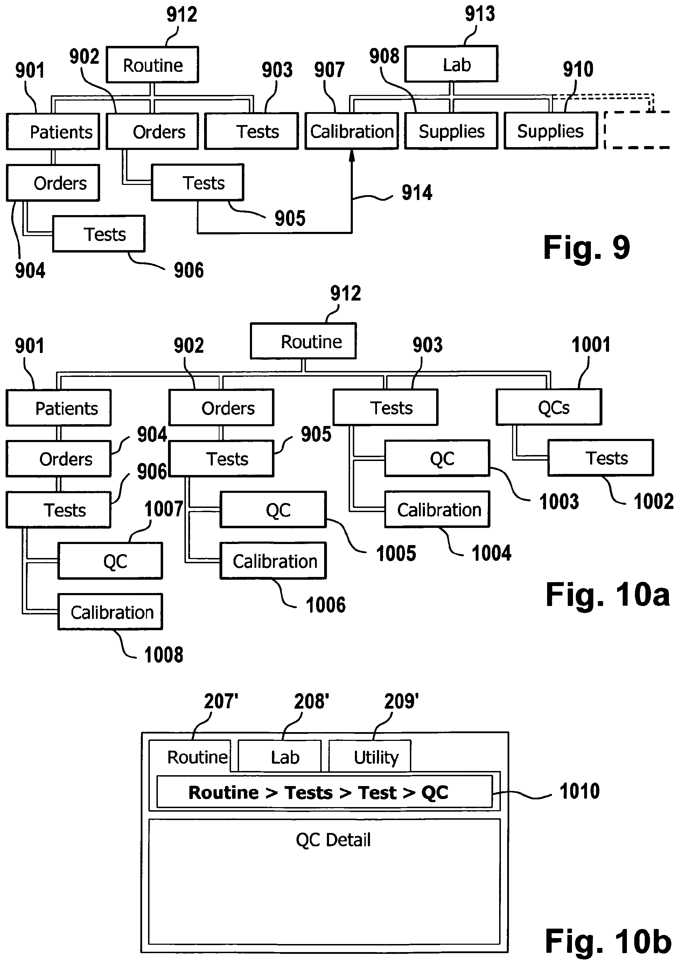

Figures 5a and 5b illustrate the execution o a drill-down analysis. Figure 5a depicts an overview on available lab-devices displayed upon selection of the 'Lab' category tab 208'. By selecting said 'Lab' category tab, the user also selects a particular drill-down graph which will be used for executing a drill-down analysis (see figure 9, drill-down graph "Lab"). The navigation path 500 indicates a user his current position within the drill-down graph. The navigation path 500 comprises a multitude of GUI elements: the selectable GUI element 530 allows a user to navigate "backward" in his chosen navigation path. The selectable GUI element 531 allows a user to navigate "forward", i.e. downwards in the selected drill-down graph provided the user already visited the selected nodes beforehand. The name of the currently selected node of the drill-down graph is displayed in a GUI element 510 at the right end of the navigation path 500. In order to allow the user to navigate to nodes the user has not yet visited, additional selectable GUI elements 502, 503, 504 are displayed which correspond to other nodes of the currently selected drill-down graph. As indicated by GUI element 510 in Figure 5a, the user has currently selected the "Lab" category drill-down graph and has selected the root node "Lab" as current node.

The selectable GUI element 532 allows a user to specify new favorites which are displayed after their specification in favorite area 401.

A user may control the operation of a particular lab-device, e.g. Cavallo, by selecting a GUI element 408 representing said device with the right mouse button and selecting the "Stop" context menu element 533. The context menu 505 comprises status information 506 of the selected lab-device Cavallo. Upon selecting context menu item 534, a user can select a node in the 'Lab' drill-down graph representing the lab-device 'Cavallo' as current node, thereby triggering the execution of a drill-down analysis based on the selected current node 'Cavallo'. As a result, device-component-IDs of logical, functional and/or physical subunits of Cavallo are used as attribute in the aggregation step being executed during the triggered drill-down analysis. Accordingly, all task data objects being represented as direct or indirect successor nodes of the selected current node 'Cavallo' and having assigned the same device-component-ID or device-component-ID range are grouped into the same task data object group.

Figure 5b depicts an aggregated view on consumables, reagents and other items used by the lab-device named 'Cavallo', the aggregated view being the result of a drill-down analysis having been executed for said lab-device by selecting the context menu item 534. The navigation path 501 comprises a set of navigation GUI elements 510, 511, 512 which specify a path in the drill-down graph starting from the root node "Lab" 913 represented by the tab 208' and ending in the currently selected current node "Cavallo" represented by GUI element 408. The lab-device pane 205 provides the logged-in user with an aggregated view on five groups of tasks which need to be executed on five logical, functional and/or physical components of Cavallo. In case a user selects a particular lab-device, e.g. Cavallo, as current node, all task data objects being grouped into the same group share at least a particular device-component-ID as attribute.

The selectable GUI elements 503, 513, 514, 516 displayed to the user correspond to other nodes of the currently selected drill-down graph. Upon selection by a user, the node represented by said selectable GUI element is used as new current node.

As a result of selecting the node representing the lab-device 'Cavallo' as current node in the 'Lab' drill-down graph, a drill-down analysis is executed and an aggregated view on task data objects having assigned Cavallo's device-ID is provided to the user as displayed in Figure 5b. The attribute used for aggregation the direct and indirect successor nodes of the 'Cavallo' node is the device-component-ID. As a result of the drill-down analysis, 5 different groups of task data objects are created and 5 GUI elements providing an aggregated view on one or more task data objects are displayed: "Reagents", "Consumables", "Samples", "Waste", and "ISE". For each of said groups of task data objects, the data contained in the task data objects belonging to said group are aggregated to generate an aggregated data value. According to the depicted embodiment, the generated data value for each of said 5 groups is indicative of whether one or more task data objects representing tasks of the highest or second highest urgency level have been found. In case at least one task data object of a task data object group has assigned a high urgency level value, the colour of the aggregation GUI element representing said group turns into the colour representing said urgency level, e.g. red for the highest urgency level or yellow for the second highest urgency level.

In figure 5a, the GUI element 408 representing Cavallo and the GUI element 409 representing Integral are displayed in yellow colour indicated by a particular hachure. Said colour indicates to a user that one or more tasks have to be executed on Cavallo and Integral in the near future (yellow represents the second highest urgency level). By selecting the node representing 'Cavallo' as current node, the user can command the server computer to provide him with a more detailed view on the tasks to be executed on Cavallo. As a result, a drill-down analysis is executed for the current node 'Cavallo' and an aggregated view on tasks to be executed in respect to five of Cavallo's logical, functional and/or physical subunits is provided to the user as shown in figure 5b. In Figure 5b, the reagents subunit GUI element 216 is displayed in yellow colour, thereby indicating that one or more tasks have to be executed which are related to the reagents compartment. By selecting said GUI element 216, the user can trigger the execution of a further drill-down analysis for the reagents subunit of Cavallo. As a result, the user is provided with a more fine-grained aggregated view on the tasks which have to be performed by or in relation to the reagents subunit (not shown).

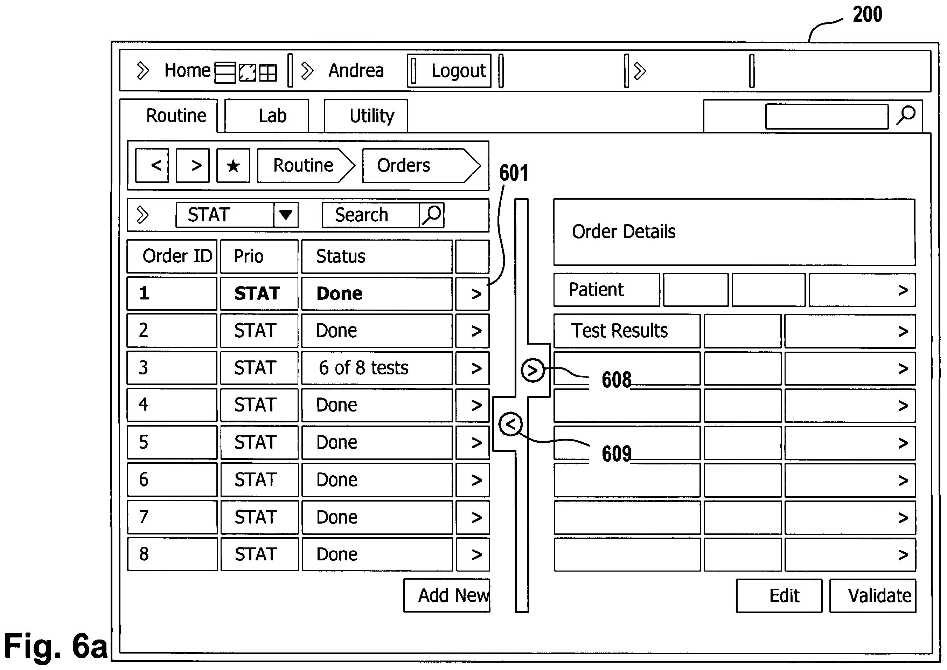

Figures 6a-6c illustrate the dynamic specification and aggregated display of tasks. The dynamic specification of new tasks in dependence on current system state allows a user working e.g. on some routine tasks to be informed immediately on a critical system state or a failure of a lab-device which requires immediate intervention by the user.

Figure 6a illustrates a graphical user interface resulting from selecting 'Routine' as current category and from selecting the node 902 'Orders' within the corresponding 'Routine' drill-down graph. On the left side of the pane a list of orders and their corresponding status is displayed. By selecting e.g. GUI element 601 corresponding to order ID 2, the details of the selected order are displayed in the 'order details' pane on the right side of the GUI and can be evaluated and verified by the user.

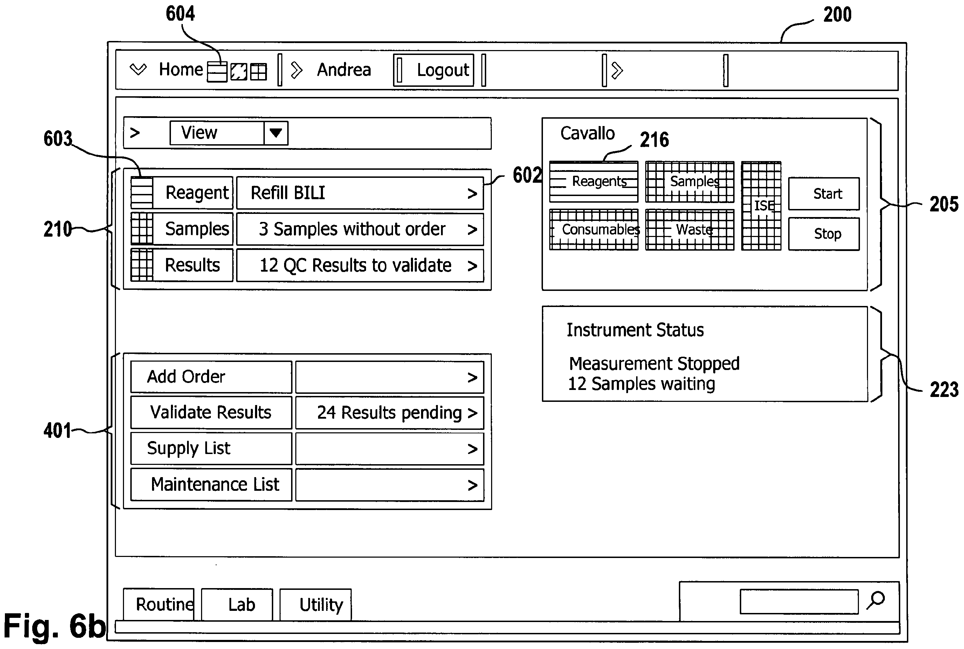

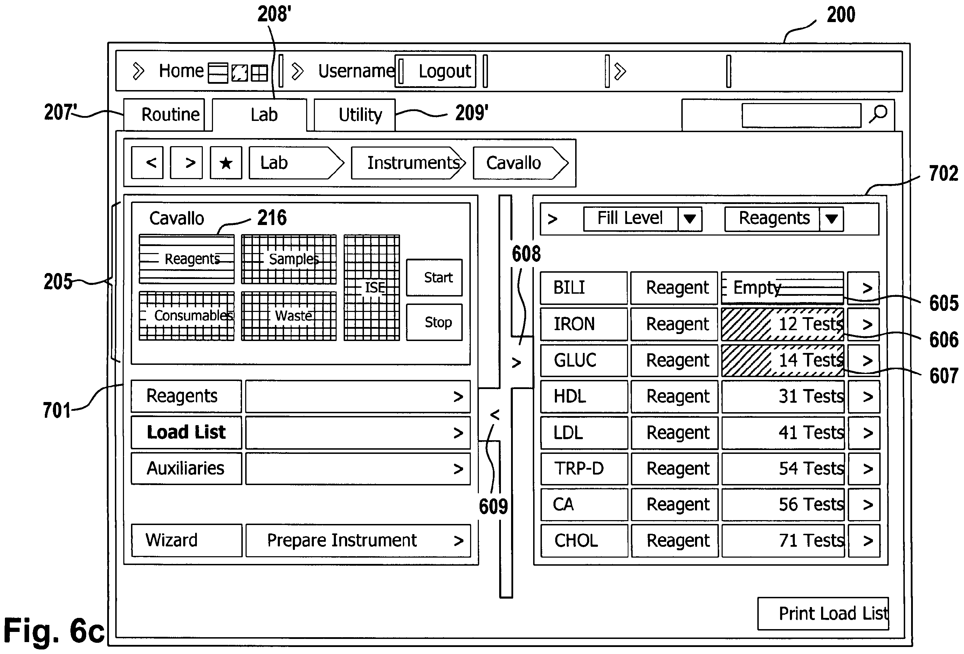

The server computer constantly receives status information of lab-devices belonging to the analysis system which are connected to the server computer. Such status information includes error codes, messages on used up consumables etc. In case a lab-device runs out of reagents, a new task data object is dynamically created, said task data object being indicative of the task to refill said reagent. Depending on the embodiment, different forms of warnings are then presented to the user, e.g. in the form of an acoustic signal and/or in the form of a refresh of the GUI, in particular of the group of squares 202. A user can navigate to the overview pane 201 which is depicted in figure 6b. The overview pane provides the user with an aggregated view of tasks having assigned a high urgency level, including the dynamically created task for refilling empty reagent bottles in Cavallo. The GUI element 602 in the tasks area represents the task of refilling a reagent required for a bilirubine analysis. By selecting GUI element 601, a user can trigger the display of data required for executing said tasks. As a result, the GUI elements contained in the lab-device area 205 are displayed. GUI element 216 in the lab-device area indicates that at least one reagent of the lab-device Cavallo needs to be refilled. GUI elements 603, 604 and 216 are displayed in red colour, thereby indicating that the reagent needs to be replaced immediately. By selecting the GUI element 216 representing the reagents subunit of Cavallo, a user can trigger the display of further details as depicted in figure 6c. The content of the lab-device area 205, in figure 6b formerly displayed on the right half of the GUI, is depicted in the detailed view in figure 6c on the left half of the GUI. In pane 704, a list of reagents currently loaded in Cavallo is displayed. The empty reagent bottle for the BILI reagent is indicated to the user in red colour of GUI element 605. Other reagents which will need to be refilled in the near future are represented as yellow coloured GUI elements 606, 607. The list of reagents displayed in pane 702 is dynamically updated.

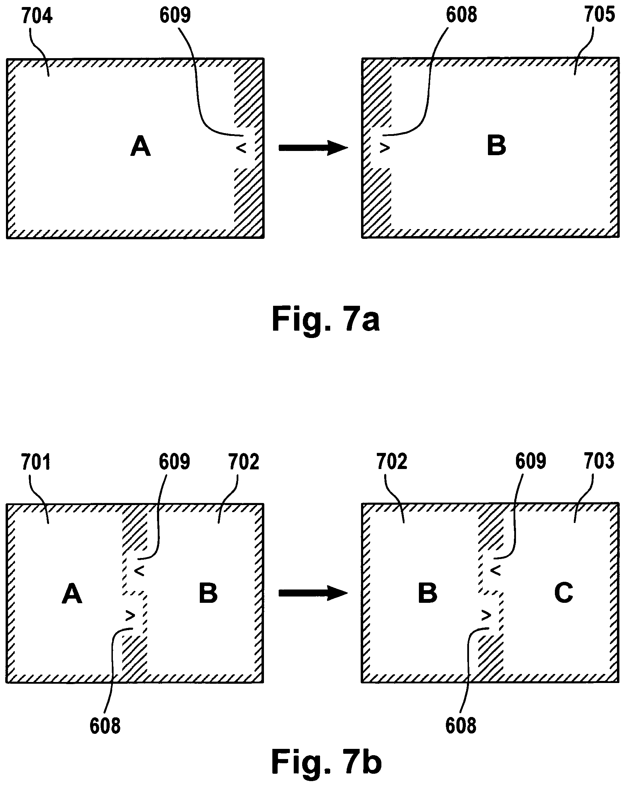

Figure 7a depicts an aggregated view generated as the result of a drill-down analysis to the user according to a first display mode. According to the first display mode, the GUI comprises a first pane 704 or a second pane 705, each of said panes being approximately of screen size. Each GUI element displayed in said first or second pane is provided according to a first GUI element version. Said version corresponds to a design specially adapted for the display of a GUI element on a small screen, in particular a screen of a mobile device. "Specially adapted" implies that the size and resolution of the displayed GUI elements allows the immediate recognition and comprehension by a user when presented to the user on a small screen. Each GUI element is a pointer to a node in the drill-down graph. At least one GUI element belonging to the first version of GUI elements is displayed in the first pane 704. Upon selection of said at least one first GUI element, the node being pointed at by the selected GUI element is used as current node and a drill-down analysis is executed for said current node. One or more second GUI elements generated as the result of said drill-down analysis are displayed in the second pane 705, the second pane being approximately of screen size. The second GUI elements are also provided according to said first GUI element version being specially adapted for displaying GUI elements on a small screen. The second pane replaces the first pane, thereby reducing the total amount of GUI elements to be displayed to the user at the same time on a small screen.

Figure 7b depicts an aggregated view according to a second display mode. According to the second display mode, the GUI comprises a first pane 701 and a second pane 702, each of said panes being approximately of half screen size. Each GUI element displayed in said first or second pane is provided according to a second GUI element version. Said version corresponds to a design specially adapted for the display of a GUI element on a medium sized screen, in particular a screen of a current computer system or touch screen of a lab-device. "Specially adapted" implies that the size and shape of the displayed GUI elements allows the immediate recognition and comprehension by a user when presented to the user on a medium-sized screen.

Each GUI element 214-220, 406-410, 430-432 is a pointer to a node in the drill-down graph (see e.g. figures 4a and 4b). At least one second version GUI element is displayed in the first pane 701, e.g. an aggregation GUI element. Upon selection of said at least one first GUI element, the node being pointed at by the selected GUI element is used as current node and a drill-down analysis is executed for said current node. One or more second GUI elements generated as the result of said drill-down analysis are displayed in the second pane 702, the second pane being approximately of half screen size. The second GUI elements are also provided according to said second GUI element version specially adapted for displaying GUI elements on a medium-sized screen. The first and the second pane are displayed to the user on the left and right side of the GUI at the same time. In case a user navigates further down in the drill-down graph, pane 702 displayed on the right side of the screen depicted in figure 6c and the left side of figure 7b will be moved to the left side of the screen, thereby replacing pane 701 while the right side of the screen displays the new pane 703.

Figure 6c corresponds to the second display mode according to which the first pane 701 and the second pane 702 are displayed simultaneously. The pagination GUI elements 608, 609 indicate that the content of the first and second pane can be changed by a user executing a drill-down analysis in a way similar to leaving through a book.

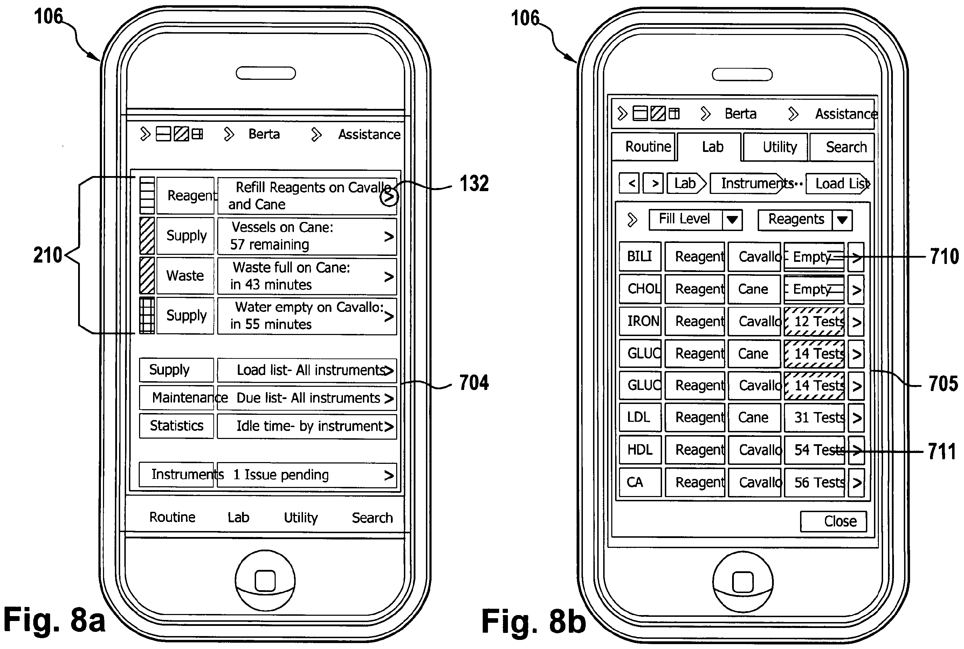

Figure 8a depicts a first pane 704 displayed on a screen of a mobile device 106 according to the first display mode. The first pane comprises the tasks pane and the favorites pane. Upon selection of the aggregation GUI element 132 corresponding to the shared task step of refilling reagents on Cavallo and Cane, a drill-down analysis is executed and the first pane 704 is replaced by the second pane 705 as displayed in figure 8b.

Figure 9 depicts parts of two different drill-down graphs corresponding to the categories 'Routine' and 'Lab', the drill-down graphs being connected via a link 914. Each box in figure 9 and 10a represents a node 901-913, 1001-1008 in a drill-down graph. Upon selection of tab 207', the drill-down graph having node 912 as root node is selected as drill-down graph. Accordingly, each of the tabs 207'-209' provide a user with different entry points for executing a drill-down analysis according to a particular graph topology, each topology corresponding to a different view of the data contained in the received task data objects added to the nodes of the drill-down graphs.

Figure 10a is a detailed illustration of the drill-down graph corresponding to the category 'Routine'. As can be seen from figure 10a, multiple drill-down analyses can be executed starting from root node 912 and ending in a "Calibration" node 1004, 1006, 1008. According to a first pathway, the "Calibration" node 1008 is reached via nodes 912, 901, 904 and 906. According to alternative second pathway, the "Calibration" node 1006 can be reached via nodes 912, 902 and 905.

Figure 10b illustrates the relation between the navigation path 500 and 1010 and the corresponding path within the drill-down graph chosen for executing a drill-down analysis. The nodes 912 and 903 belonging to the third branch of the drill-down graph depicted in figure 10a correspond to the navigation path 1010 depicted in figure 10b after a user selected a particular quality control node "QC" for executing a quality check on measurement data received during the execution of a test.

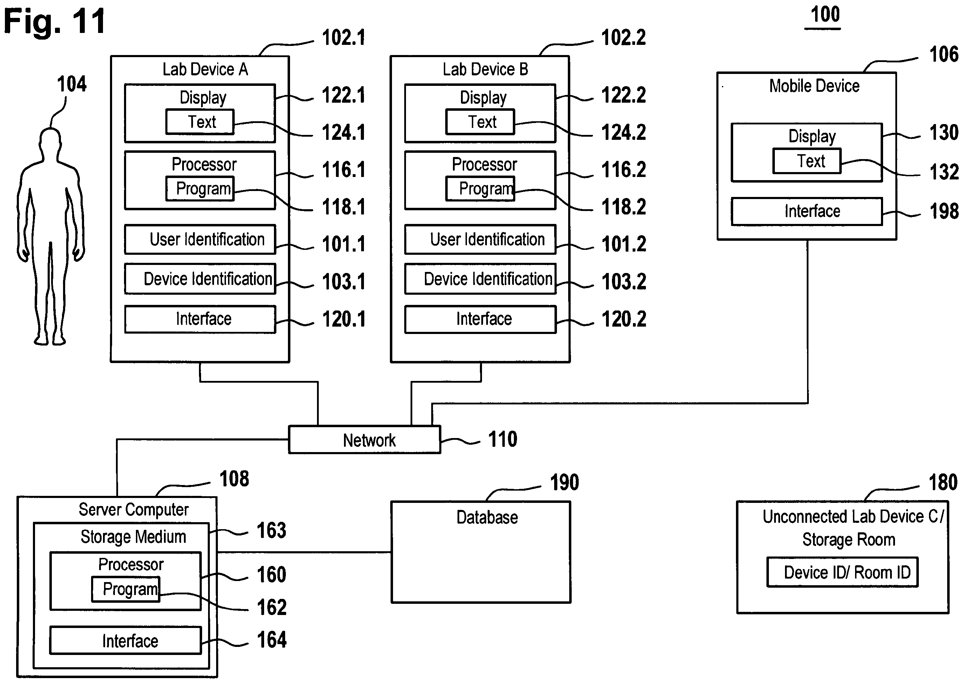

Figure 11 is a block diagram of an embodiment of an analysis system 100. The analysis system 100 comprises various lab-devices 102.1, 102.2 being coupled to a server computer 108 that controls operation of said lab-devices. According to the depicted embodiment, at least some of the lab-devices comprise a processor 116.1, 116.2 for executing computer-implemented instructions 118.1, 118.2 which allow the specification of task data objects and/or are required for exchanging data with the server computer. Depending on the implementation at least some of the lab-devices are coupled to the server computer 108 via the network 110; In the embodiment considered here the analysis system 100 has a connected lab-device A and a connected lab-device B. The connected lab-device A has at least one processor 116.1 for execution of program instructions 118.1. The connected lab-devices have a network interface 120.1, 120.2 for coupling the connected lab-device A, B to the network 110, in particular in order to enable communication with a server computer 108. The unconnected lab-device 180 can be, for example, a refrigerator, a storage room for storing reagents or samples and the like.

The connected lab-device A has a display, such as a computer monitor 122.1 for displaying a screen image 124.1 that may include a textual output. Further, the connected lab-device A has a user identification component 101.1 and a device identification component 103.1. The user identification component 101.1 serves for identification of a human user 104, such as a laboratory assistant. The user identification component 101.1 can be operable for the user's 104 entry of his or her user name and password. Alternatively or in addition the user identification component 101.1 can implement an RFID method for determining the user's 104 identity by means of an RFID chip card of that user. As an alternative or in addition the user identification component 101.1 can be operable to perform the user's 104 identification using a biometric method, such as fingerprint identification. In this instance the user identification component 101.1 comprises a sensor for sensing the user's 104 respective biometric features, such as a fingerprint sensor. The device identification component 103.1 can be a protected storage area of the connected lab-device A in which a unique device identifier for identification of the device is stored. This device identifier can be a serial number, such as a globally unique identifier (GUID) that is assigned to the connected lab-device A during its production or it can be an identifier that is unique within the analysis system and that is assigned to the connected lab-device A by an administrator of the analysis system. Alternatively the device ID is not permanently stored by the device identification component 103.1 but the device ID is computed by the device identification component 103.1 each time an identification of the connected lab-device A is required. The computation of the device ID can be executed by the device identification component 103.1 using a predefined algorithm, such as a cryptographic algorithm. The connected lab-device B of the analysis system 100 has a design that is analogous to the connected lab-device A. The connected lab-device B of the analysis system 100 has a design that is analogous to the connected lab-device A. Thus, the connected lab-device B has a user identification component 101.2, a device identification component 103.2, a processor 116.2 for execution of program instructions 118.2, a monitor 122.2 for displaying a screen image 124.2 and a metric interface 120.2 corresponding to the respective components of the connected lab-device A. The analysis system 100 in addition may comprise one or more unconnected lab-devices 180, e.g. a refrigerator for storing reagents that are required for operating the various analyzers of the analysis system 100. It is also possible to consider whole rooms, in particular storage rooms, as 'unconnected lab device'.

The server computer 108 has a processor 160 for execution of program instructions 162, and a network interface 164 for coupling the server computer 108 to the network 110. The instructions 162 are stored on a computer-readable, non-transitory storage medium, e.g. a magneto-electric data storage, a Flash memory or the like. The program instructions 162 comprise a program module for receiving, creating, modifying and/or deleting task data objects stored in a storage component 190 that is coupled to the server computer 108. In operation, the user 104 logs into the analysis system 100, e.g. via connected lab-device A. By operation of the user identification component 101.1 the user 104 is identified. The user's 104 identifier and the device identifier of the connected lab-device A provided by the device identifier component 103.1 are sent from the interface 120.1 of the connected lab-device A to the server computer 108 via the network 110. This invokes the execution of the program instructions 162 which selects and processes task data objects having assigned at least the identifier of the identified user and the device identifier received from the connected lab-device A. For example, the program 162 queries database 190 in which the task data objects are stored using the user identifier and/or the device identifier as a search term.

The signal may likewise be sent to the mobile device 106 of the user 104, whereby the GUI elements displayed to the user in this case belong to the first version of GUI elements. The mobile device 106 has an integrated display 130. Further, the mobile device 106 has a network interface 198 for coupling to the network 110. In operation, the user 104 selects one of the connected lab-devices A or B, such that the server computer 108 receives a user identifier and a device identifier. The user identifier and the device identifier are used by the program 162 for determination of one or more task data object to be executed by the user by means of the database 190.

The signal sent to the mobile device 106 of the user 104 or to any of the lab-devices can comprise information regarding the device-id or room-ID where one or more tasks have to be executed. For example, the signal can comprise a set of task data objects indicting that several reagents need to be refilled and that fresh reagent bottles can be retrieved from the unconnected lab-device C 180. Each of said task data objects comprises information on the room number where said lab-device C, in this case a refrigerator, can be found. In case multiple tasks have to be executed by the user in respect to said unconnected lab-device, the user is provided with an aggregated view on said multitude of tasks. Upon receipt of this signal by the mobile device 106 the user 104 can walk to the unconnected lab-device C for execution of the determined one or more tasks. After completion of the execution of the determined step the user 104 may use the mobile device 106 in order to acknowledge completion of the execution which is signalled from the mobile device back to the server computer 108 in order to mark the said tasks as completed in the database 190.

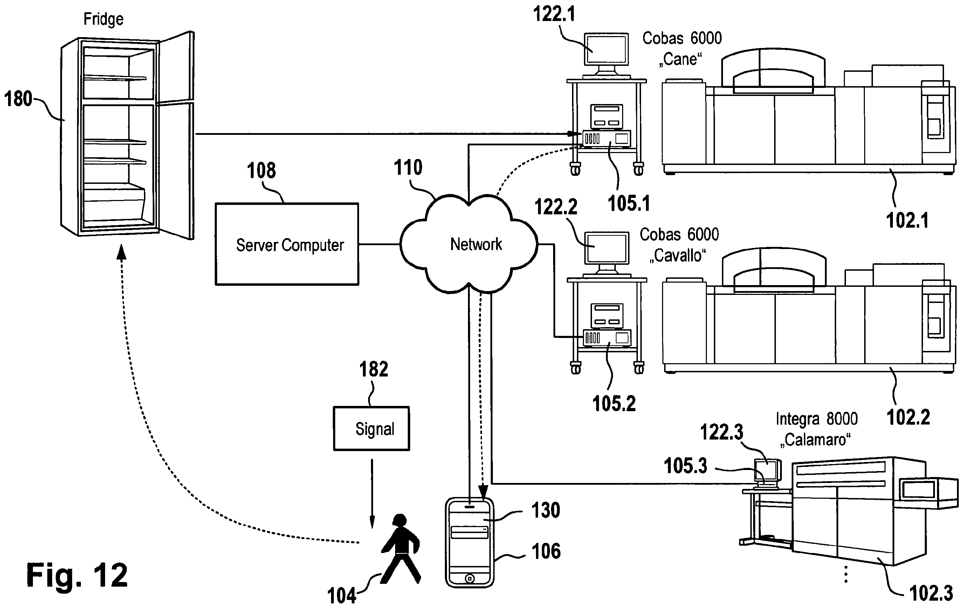

Figure 12 is a block diagram of a further embodiment of an analysis system of the invention where the requirement for refilling a reagent is signalled to a user. The depicted analysis system comprises an analyser 102.1 of the cobas 6000 type having the device ID 'Cane', analyser 102.2 of the cobas 6000 type having the device ID 'Cavallo' and analyser 102.3 of the Integra 800 type having the device ID 'Calamaro'. The analyser 102.1 and its analyser control computer 105.1 constitute connected lab-device A and the analyser 102.2 and its analyser control computer 105.2 constitute connected lab-device B. The analysis system further comprises the unconnected lab-device 180 which is a refrigerator for storing a stock of reagents.

In operation, one of the analyzers, such as the analyser 102.1, requires refilling of a reagent. For example, this is sensed by means of a sensor that is coupled to the analyser control computer 105.1. When the charging level of the reagent of the analyser 102.1 is below a predefined threshold level, the analyser control computer 105.1 generates a signal 182 that is sent to the mobile device 106, depending on the embodiment of the invention, either directly or through the intermediary of the server computer 108. According to embodiments wherein the signal 182 is sent directly from the analyser control computer 105.1 to the mobile device, the analyzer control computer is operable, in addition to the server computer, to execute the aggregating of task data objects as described for the server computer 108 or is operable to receive the results of aggregating the received task data objects from the server computer 108.

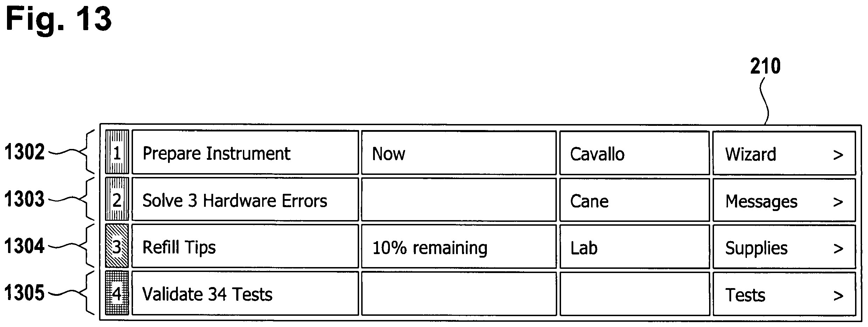

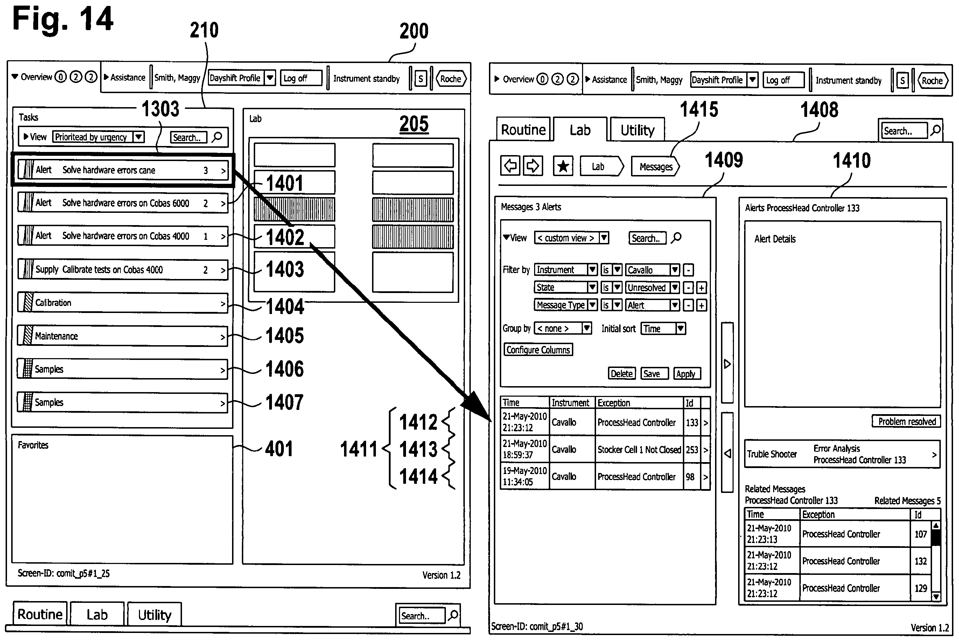

Figure 13 is a screenshot displaying 4 GUI elements 1302-1305 of 4 different aggregated task data object groups. The aggregated data value calculated for each aggregated task data object group is used for specifying each respective GUI element. For example, GUI element 1303 is indicative of the fact that 3 hardware errors on lab device 'Cane' which need to be resolved in respective tasks. GUI element 1305 is indicative of 34 test validation tasks, whereby the number '34' is the aggregated data value. Aggregation GUI element 1303 represents three task data objects for managing and resolving hardware errors, whereby the attribute used for aggregating is an identifier of messages pane 1408. The messages pane 1408 depicted in figure 14 is encoded by a particular set of program instructions which, via said messages pane, allow executing tasks related to the maintenance of said devices. Accordingly, all task data objects representing tasks related to the maintenance of lab devices which can be resolved by means of messages pane 1408 have assigned an attribute being indicative of messages pane 1408 and being used for aggregating the task data objects. In addition, the aggregation of task data objects providing the GUI elements 1303-1407 is based on a further attribute being indicative of a physical location, in this case the lab device at which a maintenance task needs to be executed. For example, GUI 1303 is indicative of maintenance tasks for the device Cane while GUI element 1401 is indicative of maintenance tasks related to the device 'Cobas 4000'. Aggregation GUI element 1304 is indicative of a plurality of supply-related tasks. All task data objects represented by GUI element 1304 have assigned an attribute being indicative of the supply pane 1505, whereby said supply pane 1505 provides a user with means for recognizing and/or resolving the aggregated supply-related tasks.

Figure 14 is a screenshot of two window panes for displaying and resolving tasks related to hardware problems of lab devices. The left window pane comprises a favorites area 401, a lab device area 205, and a tasks area 210. Each GUI element 1303-1407 is implemented as selectable aggregation GUI element and is indicative of its number of aggregated task data objects. Upon selection of one of said aggregation GUI elements, e.g. element 1303, a workflow execution view comprising message pane 1408 being suitable for executing the tasks represented by said GUI element 1303 is displayed in the right half of the screen. The function of messages pane 1408 is to provide a user with all information necessary to resolve the tasks represented by GUI element 1303. Messages pane 1408 comprises a messages area 1409, a non-aggregated task list 1411 and an alert details area 1410. The non-aggregated tasks list 1411 displays a list of tasks GUI elements 1412-1414 respectively representing singular tasks. Said tasks are collectively represented by aggregation GUI element 1303. Each task of said list 1411 comprises additional information such as the lab device having submitted the alert, the type of alert and/or the type of component of said lab device where the failure occurred, a task ID, and the time and date when the alert was submitted. The user can select one of the task GUI elements, e.g. 1412. As a result, alert details area 1410 displays additional information assisting a user in resolving the detected hardware problems.

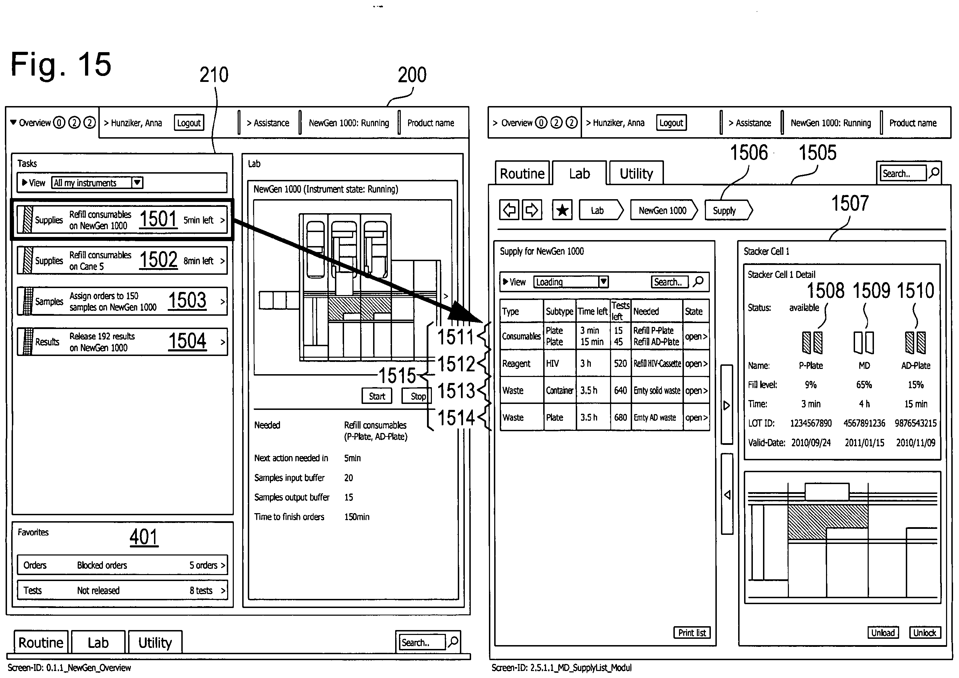

Figure 15 is a screenshot of two window panes for displaying and resolving supply related tasks. The left pane of the window comprises a tasks area 210, a lab device area 205 displaying the lab device 'NewGen 100' and its components, and a favorites area 401. Lab device area 205 comprises a list of aggregation GUI elements 1501-1504. The aggregation of task data objects is based on shared attributes which are indicative of program instructions for resolving the corresponding task. For example, program instructions specify a task execution view comprising a supply pane 1505 for assisting a user in resolving supply related tasks. The aggregation GUI elements 1501-1504 are based on a shared attribute being indicative of a physical location, in this case the lab device at which a supply task needs to be executed. For example, GUI 1501 is indicative of supply tasks for the device NewGen 1000 while GUI element 1502 is indicative of supply tasks related to the device 'Cane'. A user, by clicking on aggregation GUI element 1501, triggers the display of supply pane 1505. The supply pane comprises a non-aggregated task list 1515 and a supply status details pane 1507. The non-aggregated list comprises task GUI elements 1511-1514 respectively representing a maintenance task. A user, by selecting a particular task GUI element 1511 triggers the display of task related details in the supply status details pane 1507. The supply details area comprises GUI elements 1508-1510 indicating the supply status of components of the device NewGen 1000.

### List of reference numerals

- 100: analysis system
- 101: user identification component
- 102: lab-device
- 103: device-identification component
- 104: user
- 105: analyzer control computer
- 106: mobile user device
- 108: server computer
- 110: network
- 116: processor
- 118: program instructions
- 120: network interface
- 122: monitor
- 124: screen image
- 130: display
- 132: aggregation GUI element
- 160: processor
- 162: program instructions
- 163: storage medium
- 164: interface
- 180: unconnected lab-device/storage room
- 182: signal
- 190: database
- 198: network interface
- 200: graphical user interface
- 201: overview pane
- 202: group of squares
- 203, 204: GUI element, user logged-off
- 203', 204': GUI element, user logged-in
- 205: lab-device area
- 207-209: tabs
- 210: tasks area
- 211: colored area and
- 212: colored area
- 213: text indicating task type
- 214-215: aggregation GUI element s
- 216: GUI element for lab-device subunit "re-agents"
- 217: GUI element for lab-device subunit "consumables"
- 218: GUI element for lab-device subunit "samples"
- 219: GUI element for lab-device subunit "waste"
- 220: GUI element for lab-device subunit "ISE"
- 221: selectable GUI element "start"
- 222: selectable GUI element "stop"
- 223: lab-device status area
- 224: text indicating task type
- 251-255: step
- 300: login dialog window
- 401: favorites area
- 402-405: favorites GUI elements
- 406-410: GUI elements for lab-devices
- 430-432: aggregation GUI elements
- 500,501: navigation paths
- 502, 504: aggregation GUI elements
- 505: context menu
- 506: status information on selected device
- 507: filling level of supplies
- 510, 511, 512: navigation GUI elements
- 513, 514, 516: aggregation GUI elements
- 530-532: selectable GUI elements
- 534: context menu item
- 601: GUI element
- 602: aggregation GUI element
- 603: colored area
- 605: status information "empty reagent"
- 606: status information "12 tests remaining"
- 607: status information "14 tests remaining"
- 608-609: pagination GUI element
- 701, 704: first pane
- 702: second pane
- 703: third pane
- 705: second pane
- 912, 913: root nodes of drill-down graphs of different categories
- 901-910: nodes
- 1001-1008: nodes
- 1010: navigation path
- 1302-1305: aggregation GUI elements
- 1401-1407: aggregation GUI elements
- 1408: messages pane
- 1409: messages area
- 1410: alert details area
- 1411: non-aggregated task list
- 1412-1414: task GUI elements
- 1415: navigation GUI element "Messages"
- 1501-1504: aggregation GUI elements
- 1505: supply pane
- 1506: navigation GUI element "Supply"
- 1507: supply status details pane
- 1508-1510: aggregation GUI elements
- 1511-1514: task GUI elements
- 1515: non-aggregated task list

## Claims

1. An analysis system for analyzing biological samples comprising:
- at least one lab-device (102.1, 102.2),
- a server computer (108) having a server interface component (164) for receiving (251) task data objects, each task data object comprising at least one attribute, wherein at least some of the received task data objects are indicative of a laboratory procedure to be performed by the at least one lab-device (102.1, 102.2),
the server computer (108) comprising:
• a processing component (160) for:
- gathering and grouping (252) at least some of the received task data objects into aggregated task data object groups, wherein all task data objects belonging to the same aggregated task data object group share an attribute value or value range of the at least one attribute, the at least one shared attribute value or value range being indicative of at least one shared step of the task of said attribute's task data object, said shared step being shared by all tasks of the task data objects of said aggregated task data object group;
- specifying (253) a selectable aggregation GUI element (214-220, 1303, 1501-1504) for each of the aggregated task data object groups, said aggregation GUI element representing said aggregated task data object group;
- displaying (254) the aggregation GUI elements in an aggregated view (210), wherein the aggregated view is a view comprising at least one aggregated task data object;
- upon selection of one of the aggregation GUI elements by a user, selecting (255) the aggregated task data object group represented by the selected aggregation GUI element and automatically providing said user access to program instructions for executing the shared step of said selected aggregated task data object group; and
• a graphical user interface (122.1, 122.2, 130, 200) for
- displaying (254) the specified aggregation GUI elements for providing the aggregated view (210).

2. The analysis system according to claim 1,
• wherein a device-ID is assigned to at least some of the received task data objects, each device-ID being indicative of one of the at least one lab-devices,
• wherein the at least one lab-device comprises
∘ a device identification component (103.1, 103.2) for identifying the lab-device (102.1, 102.2) at the server computer (108) via a device-ID,
• wherein the server computer comprises
∘ a service interface component (164), wherein the service interface component is operable to send a signal (182) to the identified lab-device via the service interface component (164), the signal comprising data aggregated on the received task data objects to which is assigned the device-ID of the identified lab-device.

3. A method being implemented by an analysis system (100), the analysis system being used for processing biological samples, the method comprising the steps of:
A) receiving (251) task data objects, each task data object comprising at least one attribute, wherein at least some of the received task data objects are indicative of a laboratory procedure to be performed by the at least one lab-device (102.1, 102.2), the at least one lab-device belonging to the analysis system (100),
B) gathering and grouping (252) at least some of the received task data objects into aggregated task data object groups, wherein all task data objects belonging to the same aggregated task data object group share an attribute value or value range of the at least one attribute, the at least one shared attribute value or value range being indicative of at least one shared step of the task of said attribute's task data object, said shared step being shared by all tasks of the task data objects of said aggregated task data object group,
C) specifying (253) a selectable aggregation GUI element (214-220, 1303, 1501-1504) for each of the aggregated task data object groups, said aggregation GUI element representing said aggregated task data object group,
D) displaying (254) the aggregation GUI elements in an aggregated view on a graphical user interface (122.1, 122.2, 130, 200), wherein the aggregated view is a view comprising at least one aggregated task data object, and
E) upon selection of one of the aggregation GUI elements by a user, selecting (255) the aggregated task data object group represented by the selected aggregation GUI element and automatically providing said user access to program instructions for executing the shared step of said selected aggregated task data object group.

4. The method according to claim 3, wherein a hierarchical drill-down graph comprising at least two hierarchical levels and comprising one or more first nodes (901-9914, 1001-1008) is used for aggregating the task data objects, each first node having assigned a node attribute, the method further comprising the steps of:
- representing each received task data object as a second node of the hierarchical drill-down graph, wherein the aggregating step B comprises:
- determining one of the first nodes as current node of the hierarchical drill-down graph, the current node being a starting point for executing a drill-down analysis, the drill-down analysis being a data aggregation operation executed on the current node and all direct and indirect successor nodes of the current node, wherein when executing the aggregation operation on said nodes, the node attribute of the current node is used in said aggregation operation as aggregation attribute in accordance with step B;
- calculating, for the aggregated task data object group represented by the current node, an aggregated data value by executing a data aggregation function over all task data objects represented by any of the successor nodes of the current node.

5. The method according to claim 4, further comprising the steps of:
- storing, for each drill-down analysis, at least the aggregated data value in a drill-down history, the drill-down history being stored in a working memory,
- selecting a navigation GUI element (510-512) by a user, the user thereby selecting a new current node, the new current node corresponding to a previously executed drill-down analysis.

6. The method according to anyone of claims 3-5,
- wherein each task data object is assigned an urgency level attribute,
- wherein each aggregated task data object group is assigned an aggregated urgency level value, the aggregated urgency level value being calculated as the maximum urgency level attribute value assigned to any of the task data objects within said aggregated task data object group,
- wherein in the aggregated view selectively GUI elements of those aggregated task data object groups are displayed whose aggregated urgency level value is above a threshold.

7. The method according to anyone of claims 3-6, further comprising:
- providing each of the aggregation GUI elements according to a first aggregation GUI element version, each GUI element version corresponding to a particular graphical design, the first GUI element version being adapted to be displayed on a small screen, the small screen being a screen of a mobile user device (106), wherein each aggregation GUI element is a pointer to one of the first nodes (901-913, 915-1008) in the drill-down graph,
- displaying the aggregated view comprising the aggregation GUI elements (132) in a first pane (704), the first pane being of screen size,
- displaying a second pane (705) being of screen size, the second pane replacing the first pane, the second pane comprising:
• one or more further aggregation GUI elements, or
• one or more task GUI elements (710, 711; 1412-1414), each task GUI element representing a task data object of an aggregated task data object group represented by one of the aggregation GUI elements of the first pane, wherein upon selection of any of the task GUI elements, instructions for executing the shared step of said one aggregated task data object group are executed.

8. The method according to claim 7, wherein the content of the second pane is constructed by:
- upon selection of one of the aggregation GUI elements in the first pane, selecting one of the first nodes of the drill-down graph as current node,
- executing a drill-down analysis, the drill-down analysis being a data aggregation operation executed on the current node and all direct and indirect successor nodes of the current node, wherein when executing the aggregation operation on said nodes, the node attribute of the current node is used in said aggregation operation as aggregation attribute in accordance with step B, wherein one or more further aggregation task data object groups are created;
- representing each of the further aggregated task data object groups by one of the further aggregation GUI elements.

9. The method according to anyone of claims 3-8, the method further comprising:
- monitoring the status of the at least one lab-device to receive status information of said at least one lab-device,
- dynamically creating, modifying and/or deleting at least some of the received task data objects in dependence on the received status information, and
- triggering a re-execution of the steps B-E,
- wherein as a result of said re-execution a dynamically updated, aggregated view of tasks to be executed is provided.

10. The method according to anyone of claims 3-9,
- wherein the task data objects of the selected aggregated task data object group respectively represent a task of inspecting and/or validating a measurement result,
- wherein providing the user access to program instructions for executing the step shared by all said tasks comprises displaying a workflow-execution view, the workflow execution view comprising one or more task execution GUI elements (1410) enabling the user to inspect and/or validate said measurement results, and
- wherein the shared step of said selected aggregated task data object group is the step of automatically selecting the workflow execution view from a plurality of workflow execution views for display.

11. The method according to claim 10, the method further comprising:
- receiving, via the workflow execution view, a confirmation signal being indicative of an approval of one of the measurement results by the user;
- in case of having received the confirmation signal, automatically forwarding or making available said result to a LIS or a middleware component.

12. The method according to anyone of claims 3-8,
- wherein the at least one lab device is one of a plurality of lab devices,
- wherein the task data objects of the selected aggregated task data object group respectively represent a maintenance task of maintaining one of said plurality of lab devices,
- wherein providing the user access to program instructions for executing the step shared by all said tasks comprises displaying a workflow-execution view, the workflow execution view comprising one or more task execution GUI elements (1507), each task execution GUI element comprising information on a current state of one of the lab devices information on how to repair said lab-device.

13. The computer-implemented method of claim 12,
- wherein the shared step of said selected aggregated task data object group is selected from the group comprising:
- executing the maintenance tasks at a shared location, said shared location being shared by all lab-devices whose maintenance tasks are aggregated within the selected aggregated task data object group;
- selecting the workflow execution view from a plurality of workflow execution views for display;
- retrieving one or more consumables from a shared location for making the respective lab-device operative, said shared location being shared by all lab-devices whose maintenance tasks are aggregated within the selected aggregated task data object group;

14. The method according to anyone of claims 12-13, the method further comprising:
- receiving, via the workflow execution view, a confirmation signal being indicative of a successful maintenance by the user;
- in case the confirmation signal was received, automatically sending a command to each of the lab-devices for which the confirmation signal was received, thereby automatically activating said lab-device and enabling said lab-device to execute its respective laboratory procedure.

15. The method according to anyone of claims 3-8,
- wherein the at least one lab device is one of a plurality of lab devices,
- wherein the task data objects of the selected aggregated task data object group respectively represent a task of transporting one or more biological samples having been processed by one of the lab-devices from any of said lab-devices to a destination location,
- wherein the shared step of said selected aggregated task data object group is the step of transporting the one or more biological samples to a shared destination location,
- wherein providing the user access to program instructions for executing the shared step comprises displaying a workflow-execution view,
- automatically triggering, upon receiving via the workflow execution view a confirmation signal being indicative of a confirmation for the destination location by the user, one or more robotic units to automatically execute the confirmed transportation tasks of the selected aggregated task data object group, thereby transporting each of the biological samples having been processed by any of said lab-devices to the shared destination location.

## Patentansprüche

1. Analysesystem zum Analysieren biologischer Proben, das Folgendes aufweist:
- wenigstens ein Laborgerät (102.1, 102.2),
- einen Server-Computer (108) mit einer Serverschnittstellenkomponente (164) zum Empfangen (251) von Aufgabedatenobjekten, wobei jedes Aufgabedatenobjekt wenigstens ein Attribut aufweist, wobei wenigstens einige der empfangenen Aufgabedatenobjekte ein durch das wenigstens eine Laborgerät (102.1, 102.2) durchzuführendes Laborverfahren erkennen lassen,
wobei der Servercomputer (108) Folgendes aufweist:
• eine Verarbeitungskomponente (160) zum:
- Erfassen und Gruppieren (252) von wenigstens einigen der empfangenen Aufgabedatenobjekte in aggregierte Aufgabedatenobjektgruppen, wobei alle Aufgabedatenobjekte, die zur gleichen aggregierten Aufgabedatenobjektgruppe gehören, sich einen Attributwert oder -wertebereich des wenigstens einen Attributs teilen, wobei der wenigstens eine geteilte Attributwert oder -wertebereich wenigstens einen geteilten Schritt der Aufgabe des Aufgabedatenobjekts des genannten Attributs erkennen lässt, wobei sich alle Aufgaben der Aufgabedatenobjekte der genannten aggregierten Aufgabedatenobjektgruppe den genannten geteilten Schritt teilen;
- Vorgeben (253) eines auswählbaren Aggregations-GUI-Elements (214 - 220; 1303; 1501 - 1504) für jede der aggregierten Aufgabedatenobjektgruppen, wobei das genannte Aggregations-GUI-Element die genannte aggregierte Aufgabedatenobjektgruppe repräsentiert;
- Anzeigen (254) der Aggregations-GUI-Elemente in einer aggregierten Ansicht (210), wobei die aggregierte Ansicht eine Ansicht ist, die wenigstens ein aggregiertes Aufgabedatenobjekt aufweist;
- bei Auswahl von einem der Aggregations-GUI-Elemente durch einen Benutzer Auswählen (255) der aggregierten Aufgabedatenobjektgruppe, die durch das ausgewählte Aggregations-GUI-Element repräsentiert wird, und automatisches Gewähren, dem genannten Benutzer, von Zugang zu Programmanweisungen zum Ausführen des geteilten Schritts der genannten ausgewählten aggregierten Aufgabedatenobjektgruppe; und
• eine graphische Benutzeroberfläche (122.1, 122.2, 130, 200) zum
- Anzeigen (254) der vorgegebenen Aggregations-GUI-Elemente zum Bereitstellen der aggregierten Ansicht (210).

2. Analysesystem nach Anspruch 1,
• wobei eine Gerätekennung wenigstens einigen der empfangenen Aufgabedatenobjekte zugeordnet ist, wobei jede Gerätekennung eines der wenigstens einen Laborgeräte erkennen lässt,
• wobei das wenigstens eine Laborgerät Folgendes aufweist:
o eine Gerätekennungskomponente (103.1, 103.2) zum Identifizieren des Laborgeräts (102.1, 102.2) am Servercomputer (108) durch eine Gerätekennung,
• wobei der Servercomputer Folgendes aufweist:
∘ eine Dienstschnittstellenkomponente (164), wobei die Dienstschnittstellenkomponente zum Senden eines Signals (182) über die Dienstschnittstellenkomponente (164) an das identifizierte Laborgerät funktionell ist, wobei das Signal auf den empfangenen Aufgabedatenobjekten, denen die Gerätekennung des identifizierten Laborgeräts zugeordnet ist, aggregierte Daten aufweist.

3. Verfahren, das durch ein Analysesystem (100) implementiert wird, wobei das Analysesystem zur Bearbeitung biologischer Proben verwendet wird, wobei das Verfahren die folgenden Schritte aufweist:
A) Empfangen (251) von Aufgabedatenobjekten, wobei jedes Aufgabedatenobjekt wenigstens ein Attribut aufweist, wobei wenigstens einige der empfangenen Aufgabedatenobjekte ein durch das wenigstens eine Laborgerät (102.1, 102.2) durchzuführendes Laborverfahren erkennen lassen, wobei das wenigstens eine Laborgerät zu dem Analysesystem (100) gehört,
B) Erfassen und Gruppieren (252) von wenigstens einigen der empfangenen Aufgabedatenobjekte in aggregierte Aufgabedatenobjektgruppen, wobei alle Aufgabedatenobjekte, die zur gleichen aggregierten Aufgabedatenobjektgruppe gehören, sich einen Attributwert oder -wertebereich des wenigstens einen Attributs teilen, wobei der wenigstens eine geteilte Attributwert oder -wertebereich wenigstens einen geteilten Schritt der Aufgabe des Aufgabedatenobjekts des genannten Attributs erkennen lässt, wobei sich alle Aufgaben der Aufgabedatenobjekte der genannten aggregierten Aufgabedatenobjektgruppe den genannten geteilten Schritt teilen,
C) Vorgeben (253) eines auswählbaren Aggregations-GUI-Elements (214 - 220; 1303; 1501 - 1504) für jede der aggregierten Aufgabedatenobjektgruppen, wobei das genannte Aggregations-GUI-Element die genannte aggregierte Aufgabedatenobjektgruppe repräsentiert,
D) Anzeigen (254) der Aggregations-GUI-Elemente in einer aggregierten Ansicht auf einer graphischen Benutzeroberfläche (122.1, 122.2, 130, 200), wobei die aggregierte Ansicht eine Ansicht ist, die wenigstens ein aggregiertes Aufgabedatenobjekt aufweist, und
E) bei Auswahl von einem der Aggregations-GUI-Elemente durch einen Benutzer Auswählen (255) der aggregierten Aufgabedatenobjektgruppe, die durch das ausgewählte Aggregations-GUI-Element repräsentiert wird, und automatisches Gewähren, dem genannten Benutzer, von Zugang zu Programmanweisungen zum Ausführen des geteilten Schritts der genannten ausgewählten aggregierten Aufgabedatenobjektgruppe.

4. Verfahren nach Anspruch 3, wobei ein hierarchisches Drilldown-Diagramm, das wenigstens zwei hierarchische Ebenen aufweist und einen oder mehr erste Knoten (901 - 9914, 1001 - 1008) aufweist, zum Aggregieren der Aufgabedatenobjekte verwendet wird, wobei jedem ersten Knoten ein Knotenattribut zugeordnet ist, wobei dasVerfahren ferner die folgenden Schritte aufweist:
- Darstellen jedes empfangenen Aufgabendatenobjekts als einen zweiten Knoten des hierarchischen Drilldown-Diagramms, wobei der Aggregierungsschritt B Folgendes aufweist:
- Bestimmen von einem der ersten Knoten als aktuellen Knoten des hierarchischen Drilldown-Diagramms, wobei der aktuelle Knoten ein Ausgangspunkt zum Ausführen einer Drilldown-Analyse ist, wobei die Drilldown-Analyse eine Datenaggregationsoperation ist, die an dem aktuellen Knoten und allen direkten und indirekten Nachfolgerknoten des aktuellen Knotens ausgeführt wird, wobei beim Ausführen der Aggregationsoperation an den genannten Knoten das Knotenattribut des aktuellen Knotens in der genannten Aggregationsoperation als Aggregationsattribut gemäß Schritt B verwendet wird;
- Berechnen eines aggregierten Datenwerts für die von dem aktuellen Knoten dargestellte aggregierte Aufgabedatenobjektgruppe durch Ausführen einer Datenaggregationsfunktion über alle Aufgabedatenobjekte, die von einem der Nachfolgerknoten des aktuellen Knotens dargestellt werden.

5. Verfahren nach Anspruch 4, das ferner die folgenden Schritte aufweist:
- für jede Drilldown-Analyse Speichern wenigstens des aggregierten Datenwerts in einer Drilldown-Historie, wobei die Drilldown-Historie in einem Arbeitsspeicher gespeichert wird,
- Auswählen eines Navigations-GUI-Elements (510 - 512) durch einen Benutzer, wobei der Benutzer dadurch einen neuen aktuellen Knoten auswählt, wobei der neue aktuelle Knoten einer zuvor ausgeführten Drilldown-Analyse entspricht.

6. Verfahren nach einem der Ansprüche 3 bis 5,
- wobei jedem Aufgabendatenobjekt ein Dringlichkeitsgradattribut zugeordnet wird,
- wobei jeder aggregierten Aufgabedatenobjektgruppe ein aggregierter Dringlichkeitsgradwert zugeordnet wird, wobei der aggregierte Dringlichkeitsgradwert als der maximale irgendeinem der Aufgabedatenobjekte in der genannten aggregierten Aufgabedatenobjektgruppe zugeordnete Dringlichkeitsgradattributwert berechnet wird,
- wobei in der aggregierten Ansicht selektiv GUI-Elemente jener aggregierten Aufgabedatenobjektgruppen angezeigt werden, deren aggregierter Dringlichkeitsgradwert über einem Schwellenwert liegt.

7. Verfahren nach einem der Ansprüche 3 bis 6, das ferner Folgendes aufweist:
- Bereitstellen von jedem der Aggregations-GUI-Elemente gemäß einer ersten Aggregations-GUI-Elementeversion, wobei jede GUI-Elementeversion einer speziellen graphischen Gestaltung entspricht, wobei die erste GUI-Elementeversion zum Anzeigen auf einem kleinen Bildschirm ausgeführt ist, wobei der kleine Bildschirm ein Bildschirm einer Mobil-Benutzervorrichtung (106) ist, wobei jedes Aggregations-GUI-Element ein Zeiger auf einen der ersten Knoten (901 - 913, 915 - 1008) im Drilldown-Diagramm ist,
- Anzeigen der aggregierten Ansicht, die die Aggregations-GUI-Elemente (132) aufweist, in einem ersten Fenster (704), wobei das erste Fenster Bildschirmgröße hat,
- Anzeigen eines zweiten Fensters (705), das Bildschirmgröße hat, wobei das zweite Fenster das erste Fenster ersetzt, wobei das zweite Fenster Folgendes aufweist:
• ein oder mehr weitere Aggregations-GUI-Elemente oder
• ein oder mehr Aufgabe-GUI-Elemente (710, 711; 1412 - 1414), wobei jedes Aufgabe-GUI-Element ein Aufgabedatenobjekt einer aggregierten Aufgabedatenobjektgruppe darstellt, die durch eines der Aggregations-GUI-Elemente des ersten Fensters dargestellt wird, wobei bei Auswahl von einem der Aufgabe-GUI-Elemente Anweisungen zum Ausführen des geteilten Schritts der genannten einen aggregierten Aufgabedatenobjektgruppe ausgeführt werden.

8. Verfahren nach Anspruch 7, wobei der Inhalt des zweiten Fensters konstruiert wird durch:
- bei Auswahl von einem der Aggregations-GUI-Elemente im ersten Fenster Auswählen von einem der ersten Knoten des Drilldown-Diagramms als aktuellen Knoten,
- Ausführen einer Drilldown-Analyse, wobei die Drilldown-Analyse eine Datenaggregationsoperation ist, die an dem aktuellen Knoten und allen direkten und indirekten Nachfolgerknoten des aktuellen Knotens ausgeführt wird, wobei beim Ausführen der Aggregationsoperation an den genannten Knoten das Knotenattribut des aktuellen Knotens in der genannten Aggregationsoperation als Aggregationsattribut gemäß Schritt B verwendet wird, wobei eine oder mehr weitere Aggregationsaufgabedatenobjektgruppen erstellt werden;
- Darstellen von jeder der weiteren aggregierten Aufgabedatenobjektgruppen durch eines der weiteren Aggregations-GUI-Elemente.

9. Verfahren nach einem der Ansprüche 3 bis 8, wobei das Verfahren ferner Folgendes aufweist:
- Überwachen des Status des wenigstens einen Laborgeräts zum Empfangen von Statusinformationen über das genannte wenigstens eine Laborgerät,
- dynamisches Erstellen, Modifizieren und/oder Löschen von wenigstens einigen der empfangenen Aufgabedatenobjekte in Abhängigkeit von den empfangenen Statusinformationen und
- Auslösen einer erneuten Ausführung der Schritte B bis E,
- wobei infolge der genannten erneuten Ausführung eine dynamisch aktualisierte aggregierte Ansicht von auszuführenden Aufgaben bereitgestellt wird.

10. Verfahren nach einem der Ansprüche 3 bis 9,
- wobei die Aufgabedatenobjekte der ausgewählten aggregierten Aufgabedatenobjektgruppe jeweils eine Aufgabe des Prüfens und/oder Validierens eines Messergebnisses darstellen,
- wobei das Gewähren, dem Benutzer, von Zugang zu Programmanweisungen zum Ausführen des Schritts, den sich alle genannten Aufgaben teilen, das Anzeigen einer Arbeitsablauf-Ausführungsansicht aufweist, wobei die Arbeitsablauf-Ausführungsansicht ein oder mehr Aufgabenausführungs-GUI-Elemente (1410) aufweist, die den Benutzer zum Prüfen und/oder Validieren der genannten Messergebnisse ermöglichen, und
- wobei der geteilte Schritt der genannten ausgewählten aggregierten Aufgabedatenobjektgruppe der Schritt des automatischen Auswählens der Arbeitsablauf-Ausführungsansicht aus einer Mehrzahl von Arbeitsablauf-Ausführungsansichten zur Anzeige ist.

11. Verfahren nach Anspruch 10, wobei das Verfahren ferner Folgendes aufweist:
- Empfangen eines Bestätigungssignals über die Arbeitsablauf-Ausführungsansicht, das eine Abnahme von einem der Messergebnisse durch den Benutzer erkennen lässt;
- im Fall, dass das Bestätigungssignal empfangen wurde, automatisches Weiterleiten oder Verfügbarmachen des genannten Ergebnisses an bzw. für ein LIS oder eine Middleware-Komponente.

12. Verfahren nach einem der Ansprüche 3 bis 8,
- wobei das wenigstens eine Laborgerät eines von einer Mehrzahl von Laborgeräten ist,
- wobei die Aufgabedatenobjekte der ausgewählten aggregierten Aufgabedatenobjektgruppe jeweils eine Instandhaltungsaufgabe des Instandhaltens von einem der genannten Mehrzahl von Laborgeräten darstellen,
- wobei das Gewähren, dem Benutzer, von Zugang zu Programmanweisungen zum Ausführen des Schritts, den sich alle genannten Aufgaben teilen, das Anzeigen einer Arbeitsablauf-Ausführungsansicht aufweist, wobei die Arbeitsablauf-Ausführungsansicht ein oder mehr Aufgabenausführungs-GUI-Elemente (1507) aufweist, wobei jedes Aufgabeausführungs-GUI-Element Informationen über einen aktuellen Zustand von einem der Laborgeräte und/oder Informationen darüber, wie das genannte Laborgerät zu reparieren ist, aufweist.

13. Computerimplementiertes Verfahren nach Anspruch 12,
- wobei der geteilte Schritt der genannten ausgewählten aggregierten Aufgabedatenobjektgruppe aus der Gruppe ausgewählt wird, die Folgendes aufweist:
- Ausführen der Instandhaltungsaufgaben an einem gemeinsam genutzten Ort, wobei sich alle Laborgeräte, deren Instandhaltungsaufgaben in der ausgewählten aggregierten Aufgabedatenobjektgruppe aggregiert werden, den genannten gemeinsam genutzten Ort teilen;
- Auswählen der Arbeitsablauf-Ausführungsansicht aus einer Mehrzahl von Arbeitsablauf-Ausführungsansichten zur Anzeige;
- Abrufen von einem oder mehr Verbrauchsmaterialien von einem gemeinsam genutzten Ort zum Betriebsfähigmachen des jeweiligen Laborgeräts, wobei sich alle die Laborgeräte den genannten gemeinsam genutzten Ort teilen, deren Instandhaltungsaufgaben in der ausgewählten aggregierten Aufgabedatenobjektgruppe aggregiert werden.

14. Verfahren nach einem der Ansprüche 12 bis 13, wobei das Verfahren ferner Folgendes aufweist:
- Empfangen eines Bestätigungssignals über die Arbeitsablauf-Ausführungsansicht, das eine erfolgreiche Instandhaltung durch den Benutzer erkennen lässt;
- im Fall, dass das Bestätigungssignal empfangen wurde, automatisches Senden eines Befehls an jedes der Laborgeräte, für die ein Bestätigungssignal empfangen wurde, wodurch das genannte Laborgerät automatisch aktiviert wird und das genannte Laborgerät befähigt wird, sein jeweiliges Laborverfahren auszuführen.

15. Verfahren nach einem der Ansprüche 3 bis 8,
- wobei das wenigstens eine Laborgerät eines von einer Mehrzahl von Laborgeräten ist,
- wobei die Aufgabedatenobjekte der ausgewählten aggregierten Aufgabedatenobjektgruppe jeweils eine Aufgabe des Transportierens von einer oder mehr biologischen Proben, die von einem der Laborgeräte bearbeitet wurden, von einem der genannten Laborgeräte zu einem Zielort darstellen,
- wobei der geteilte Schritt der genannten ausgewählten aggregierten Aufgabedatenobjektgruppe der Schritt des Transportierens der einen oder mehr biologischen Proben an einen geteilten Zielort ist,
- wobei das Gewähren, dem Benutzer, von Zugang zu Programmanweisungen zum Ausführen des geteilten Schritts das Anzeigen einer Arbeitsablauf-Ausführungsansicht aufweist,
- bei Empfang eines Bestätigungssignals über die Arbeitsablauf-Ausführungsansicht, das eine Bestätigung für den Zielort erkennen lässt, durch den Benutzer automatisches Auslösen von einer oder mehr Robotereinheiten zum automatischen Ausführen der bestätigten Transportaufgaben der ausgewählten aggregierten Aufgabedatenobjektgruppe, wodurch jede der biologischen Proben, die von einem der genannten Laborgeräte bearbeitet wurde, an den geteilten Zielort transportiert wird.

## Revendications

1. Système d'analyse pour analyser des échantillons biologiques comprenant :
- au moins un dispositif de laboratoire (102.1, 102.2),
- un ordinateur serveur (108) ayant un composant d'interface de serveur (164) pour recevoir (251) des objets de données de tâches, chaque objet de données de tâche comprenant au moins un attribut, où au moins certains des objets de données de tâches reçus sont indicateurs d'une procédure de laboratoire devant être exécutée par l'au moins un dispositif de laboratoire (102.1, 102.2),
l'ordinateur serveur (108) comprenant :
• un composant de traitement (160) pour :
- rassembler et regrouper (252) au moins certains des objets de données de tâches reçus en groupes d'objets de données de tâches agrégés, où tous les objets de données de tâches faisant partie du même groupe d'objets de données de tâches agrégés partagent une valeur d'attribut ou une plage de valeurs de l'au moins un attribut, l'au moins une valeur d'attribut, ou plage de valeurs partagée, étant indicatrice d'au moins une étape partagée de la tâche dudit objet de données de tâche de l'attribut, ladite étape partagée étant partagée par toutes les tâches des objets de données de tâches dudit groupe d'objets de données de tâches agrégés ;
- spécifier (253) un élément d'interface d'utilisateur graphique GUI d'agrégation (214, 220, 1303, 1501 à 1504) pouvant être sélectionné pour chacun des groupes d'objets de données de tâches agrégés, ledit élément de GUI d'agrégation représentant ledit groupe d'objets de données de tâches agrégés ;
- afficher (254) les éléments de GUI d'agrégation dans une vue agrégée (210), où la vue agrégée est une vue comprenant au moins un objet de données de tâche agrégé ;
- lors de la sélection de l'un des éléments de GUI d'agrégation par un utilisateur, sélectionner (225) le groupe d'objets de données de tâches agrégés représenté par l'élément de GUI d'agrégation sélectionné et fournir automatiquement ledit accès d'utilisateur à des instructions de programme pour l'exécution de l'étape partagée dudit groupe d'objets de données de tâches agrégés choisi ; et
• une interface d'utilisateur graphique (122.1, 122.2, 130, 200) pour
- afficher (254) les éléments de GUI d'agrégation spécifiés pour fournir la vue agrégée (210).

2. Système d'analyse selon la revendication 1,
• dans lequel un identifiant de dispositif est associé à au moins certains des objets de données de tâches reçus, chaque identifiant de dispositif étant indicateur d'au moins un des dispositifs de laboratoire,
• dans lequel, l'au moins un dispositif de laboratoire comprend
∘ un composant d'identification de dispositif (103.1, 103.2) pour identifier le dispositif de laboratoire (102.1, 102.2) au niveau de l'ordinateur serveur (108) par le biais d'un identifiant de dispositif,
• dans lequel l'ordinateur serveur comprend
∘ un composant d'interface de service (164), où le composant d'interface de service peut fonctionner pour envoyer un signal (182) au dispositif de laboratoire identifié par le biais du composant d'interface de service (164), le signal comprenant des données agrégées concernant les objets de données de tâches reçus auxquels est associé l'identifiant de dispositif du dispositif de laboratoire identifié.

3. Procédé étant mis en oeuvre par un système d'analyse (100), le système d'analyse étant utilisé pour traiter des échantillons biologiques, le procédé comprenant les étapes :
A) de réception (251) d'objets de données de tâches, chaque objet de données de tâches comprenant au moins un attribut, où au moins certains des objets de données de tâches reçus sont indicateurs d'une procédure de laboratoire devant être exécutée par l'au moins un dispositif de laboratoire (102.1, 102.2), l'au moins un dispositif de laboratoire faisant partie du système d'analyse (100),
B) de rassemblement et de regroupement (252) d'au moins certains des objets de données de tâches reçus en groupes d'objets de données de tâches agrégés, où tous les objets de données de tâches faisant partie du même groupe d'objets de données de tâches agrégés partagent une valeur d'attribut ou une plage de valeurs de l'au moins un attribut, l'au moins une valeur d'attribut ou plage de valeurs partagée étant indicatrice d'au moins une étape partagée de la tâche dudit objet de données de tâche de l'attribut, ladite étape partagée étant partagée par toutes les tâches des objets de données de tâches dudit groupe d'objets de données de tâches agrégés,
C) de spécification (253) d'un élément de GUI d'agrégation (214 à 220, 1303, 1501 à 1504) pouvant être sélectionné pour chacun des groupes d'objets de données de tâches agrégés, ledit élément de GUI d'agrégation représentant ledit groupe d'objets de données de tâches agrégés,
D) d'affichage (254) des éléments de GUI d'agrégation dans une vue agrégée sur une interface d'utilisateur graphique (122.1, 122.2, 130, 200), où la vue agrégée est une vue comprenant au moins un objet de données de tâche agrégé, et
E) lors de la sélection de l'un des éléments de GUI d'agrégation par un utilisateur, de sélection (225) du groupe d'objets de données de tâches agrégés représenté par l'élément de GUI d'agrégation sélectionné et de fourniture automatique dudit accès d'utilisateur à des instructions de programme pour l'exécution de l'étape partagée dudit groupe d'objets de données de tâches agrégés choisi.

4. Procédé selon la revendication 3, dans lequel un graphe détaillé hiérarchique comprenant au moins deux niveaux hiérarchiques et comprenant un ou plusieurs premiers noeuds (901 à 9914, 1001 à 1008) est utilisé pour agréger les objets de données de tâches, chaque premier noeud se voyant associé un attribut de noeud, le procédé comprenant en outre les étapes :
- de représentation de chaque objet de données de tâches reçu en tant que deuxième noeud du graphe détaillé hiérarchique, où l'étape B d'agrégation comprend :
- la détermination de l'un des premiers noeuds en tant que noeud considéré du graphe détaillé hiérarchique, le noeud considéré étant un point de départ pour l'exécution d'une analyse détaillée, l'analyse détaillée étant une opération d'agrégation de données exécutée sur le noeud considéré et tous les noeuds successeurs directs et indirects du noeud considéré, où, lors de l'exécution de l'opération d'agrégation sur lesdits noeuds, l'attribut de noeud du noeud considéré est utilisé dans ladite opération d'agrégation en tant qu'attribut d'agrégation en accord avec l'étape B ;
- le calcul, pour le groupe d'objets de données de tâches agrégé représenté par le noeud considéré, d'une valeur de données agrégée en exécutant une fonction d'agrégation de données sur tous les objets de données de tâches représentés par l'un quelconque des noeuds successeurs du noeud considéré.

5. Procédé selon la revendication 4, comprenant en outre les étapes :
- de stockage, pour chaque analyse détaillée, d'au moins une valeur de donnée agrégée dans un historique détaillé, l'historique détaillé étant stocké dans une mémoire de travail,
- de sélection d'un élément de GUI de navigation (510-512) par un utilisateur, l'utilisateur sélectionnant ainsi un nouveau noeud considéré, le nouveau noeud considéré correspondant à une analyse détaillée exécutée précédemment.

6. Procédé selon l'une quelconque des revendications 3 à 5,
- dans lequel chaque objet de données de tâche est associé à un attribut de niveau d'urgence,
- dans lequel, chaque groupe d'objets de données de tâches agrégés est associé à une valeur de niveau d'urgence agrégée, la valeur de niveau d'urgence agrégée étant calculée comme la valeur d'attribut de niveau d'urgence maximale associée à l'un quelconque des objets de données de tâches dans ledit groupe d'objets de données de tâches agrégés,
- dans lequel, dans la vue agrégée des éléments de GUI de ces groupes d'objets de données de tâches agrégés, sont affichés ceux dont la valeur de niveau d'urgence agrégé est au-dessus d'un seuil.

7. Procédé selon l'une quelconque des revendications 3 à 6, comprenant en outre
- la fourniture de chacun des éléments de GUI d'agrégation en fonction d'une première version d'élément de GUI d'agrégation, chaque version d'élément correspondant à une conception graphique particulière, la première version d'élément de GUI étant adaptée pour être affichée sur un petit écran, le petit écran étant un écran adapté d'un dispositif d'utilisateur mobile (106), où chaque élément de GUI d'agrégation est un pointeur pour un des premiers noeuds (901 à 913, 915 à 1008) dans le graphe détaillé,
- l'affichage de la vue agrégée comprenant les éléments de GUI d'agrégation (132) dans un premier panneau (704), le premier panneau étant de la taille d'un écran,
- l'affichage d'un second panneau (705) étant de la taille d'un écran, le second panneau remplaçant le premier panneau, le second panneau comprenant :
• un ou plusieurs autres éléments de GUI d'agrégation, ou
• un ou plusieurs éléments de GUI de tâches (710, 711 ; 1412 à 1414), chaque élément de GUI de tâche représentant un objet de données de tâche d'un groupe d'objets de données de tâches agrégés représenté par l'un des éléments de GUI d'agrégation du premier panneau, où, lors de la sélection de l'un quelconque des éléments de GUI de tâches, des instructions pour l'exécution de l'étape partagée dudit groupe d'objets de données de tâches agrégés sont exécutées.

8. Procédé selon la revendication 7, dans lequel le contenu du second panneau est constitué par :
- lors de la sélection de l'un des éléments de GUI d'agrégation dans le premier panneau, la sélection d'un des premiers noeuds du graphe détaillé en tant que noeud considéré,
- l'exécution d'une analyse détaillée, l'analyse détaillée étant une opération d'agrégation de données exécutée sur le noeud considéré et tous les noeuds successeurs directs et indirects du noeud considéré, où, lors de l'exécution de l'opération d'agrégation sur lesdits noeuds, l'attribut de noeud du noeud considéré est utilisé dans ladite opération d'agrégation en tant qu'attribut d'agrégation en accord avec l'étape B, où un ou plusieurs autres groupes d'objets de tâches d'agrégation sont créés ;
- la représentation de chacun des autres groupes d'objets de données de tâches agrégés par un des autres éléments de GUI d'agrégation.

9. Procédé selon l'une quelconque des revendications 3 à 8, le procédé comprenant en outre :
- la surveillance de l'état de l'au moins un dispositif de laboratoire pour recevoir des informations sur l'état d'au moins un dispositif de laboratoire,
- la création dynamique, la modification et/ou l'effacement d'au moins certains des objets de données de tâches reçus en fonction de l'information sur l'état reçue, et
- le déclenchement d'une réexécution des étapes B à E,
- où, en tant que résultat de la dite réexécution, une vue agrégée de tâches devant être exécutées mise à jour de manière dynamique est fournie.

10. Procédé selon l'une quelconque des revendications 3 à 9,
- dans lequel les objets de données de tâches du groupe d'objets de données de tâches agrégés sélectionnés représentent respectivement une tâche d'inspection et/ou de validation d'un résultat de mesure,
- dans lequel la fourniture de l'accès d'utilisateur aux instructions de programme pour l'exécution de l'étape partagée par toutes lesdites tâches comprend l'affichage d'une vue d'exécution d'un flux des travaux, la vue d'exécution du flux des travaux comprenant un ou plieurs éléments de GUI d'exécution de tâches (1410) permettant à l'utilisateur d'inspecter et/ou de valider lesdits résultats de mesure, et
- dans lequel l'étape partagée dudit groupe d'objets de données de tâche agrégés choisi est l'étape de sélection automatique de la vue d'exécution du flux des travaux à partir d'une pluralité de vues d'exécution de flux des travaux pour affichage.

11. Procédé selon la revendication 10, le procédé comprenant en outre :
- la réception, par le biais de la vue d'exécution du flux des travaux, d'un signal de confirmation étant indicateur d'une approbation de l'un des résultats de mesure par l'utilisateur ;
- dans le cas de la réception du signal de confirmation, la transmission de manière automatique ou la mise en disponibilité dudit résultat au niveau d'un LIS ou d'un composant d'intergiciel.

12. Procédé selon l'une quelconque des revendications 3 à 8,
- dans lequel l'au moins un dispositif de laboratoire est un dispositif d'une pluralité de dispositifs de laboratoire,
- dans lequel les objets de données de tâches du groupe d'objets de données de tâches agrégés sélectionné représentent respectivement une tâche de maintenance d'entretien d'un de ladite pluralité des dispositifs de laboratoire,
- dans lequel, la fourniture de l'accès d'utilisateur aux instructions de programme pour l'exécution de l'étape partagée par toutes lesdites tâches comprend l'affichage de la vue d'exécution du flux des travaux, la vue d'exécution du flux des travaux comprenant un ou plusieurs éléments de GUI d'exécution de tâche (1507), chaque élément de GUI d'exécution de tâche comprenant une information sur un état en cours de l'un des dispositifs de laboratoire et/ou une information concernant la manière de réparer ledit dispositif de laboratoire.

13. Procédé mis en oeuvre par ordinateur selon la revendication 12,
- dans lequel l'étape partagée dudit groupe d'objets de données de tâches agrégés sélectionné est choisie à partir du groupe comprenant :
- l'exécution des tâches de maintenance au niveau d'un lieu partagé, ledit lieu partagé étant partagé par tous les dispositifs de laboratoire dont les tâches de maintenance sont agrégées dans le groupe d'objets de données de tâches agrégés choisi ;
- la sélection de la vue d'exécution du flux des travaux à partir d'une pluralité de vues d'exécution de flux des travaux pour l'affichage ;
- la récupération d'un ou de plusieurs consommables à partir d'un lieu partagé pour rendre le dispositif de laboratoire respectif opérationnel, ledit lieu partagé étant partagé par tous les dispositifs de laboratoire dont les tâches de maintenance sont agrégées dans le groupe d'objets de données de tâches agrégés choisi.

14. Procédé selon l'une quelconque des revendications 12 ou 13, le procédé comprenant en outre :
- la réception, par le biais de la vue d'exécution du flux des travaux, d'un signal de confirmation étant indicateur d'une maintenance réussie par l'utilisateur ;
- dans le cas de la réception du signal de confirmation, l'envoi automatique d'une commande à chacun des dispositifs de laboratoire pour lesquels un signal d'information a été reçu, activant ainsi de manière automatique ledit dispositif de laboratoire et permettant audit dispositif de laboratoire d'exécuter sa procédure de laboratoire respective.

15. Procédé selon l'une quelconque des revendications 3 à 8,
- dans lequel l'au moins un dispositif de laboratoire est un dispositif d'une pluralité de dispositifs de laboratoire,
- dans lequel les objets de données de tâches du groupe d'objets de données de tâches agrégés choisi représentent respectivement une tâche de transport d'un ou de plusieurs échantillons biologiques ayant été traités par l'un des dispositifs de laboratoire à partir de l'un quelconque desdits dispositifs de laboratoire vers un lieu de destination,
- dans lequel l'étape partagée dudit groupe d'objets de données de tâches agrégés choisi est l'étape de transport du ou des échantillons biologiques vers un lieu de destination partagé,
- dans lequel la fourniture de l'accès d'utilisateur aux instructions de programme pour l'exécution de l'étape partagée comprend l'affichage d'une vue d'exécution de flux des travaux,
- le déclenchement de manière automatique lors de la réception, par le biais de la vue d'exécution du flux des travaux, d'un signal de confirmation étant indicateur d'une confirmation pour le lieu de destination par l'utilisateur, d'une ou de plusieurs unités robotiques pour exécuter de manière automatique les tâches de transport confirmées du groupe d'objets de données de tâches agrégés choisi, transportant ainsi chacun des échantillons biologiques ayant été traité par l'un quelconque des dispositifs de laboratoire vers le lieu de destination partagé.
